# EUROPEAN PATENT APPLICATION

(11) **EP 4 371 985 A1**
(43) Date of publication of application: **22.05.2024**
(21) Application number: 22856212.0
(22) Date of filing: 10.08.2022
(51) Int. Cl.: C07D 403/14, C07D 207/38, A61K 31/409

(54) **METHOD FOR SYNTHESIZING BILIRUBIN**

(30) Priority: 11.08.2021 KR 20210106106
(71) Applicant: Bilix Co., Ltd., Seoul 06241 (KR)
(72) Inventor: KIM, Myung Lip, Busan 48272 (KR); MA, Sang Ho, Suwon-si, Gyeonggi-do 16438 (KR); PARK, Ki Soo, Suwon-si, Gyeonggi-do 16222 (KR); JUN, Hee Goo, Suwon-si, Gyeonggi-do 16508 (KR); KIM, Da Eun, Yongin-si, Gyeonggi-do 16944 (KR)
(74) Representative: BCKIP Part mbB
(86) International application number: PCT/KR2022/011913
(87) International publication number: WO 2023/018215

(57) **Abstract**

The present invention relates to a novel synthesis method of bilirubin. The method includes preparing a compound represented by Formula 3 by coupling a compound represented by Formula 1 with a compound represented by Formula 2, thereby firstly chemically synthesizing bilirubin and PEGylated bilirubin, which are usefully used in a medical product or the like.

## Description

### [Technical Field]

The present invention relates to a novel synthesis method of bilirubin.

### [Background Art]

Bilirubin is a component of bile and is mostly generated from hemoglobin in the body. Bilirubin is a yellowish final metabolite formed from heme, and despite many hydrophilic groups, it is extremely hydrophobic due to intramolecular hydrogen bonding.

Bilirubin was considered an undesirable substance as it caused jaundice when the blood level was high. However, in a recently published study, it was found that a slightly higher blood concentration of bilirubin significantly lowered the possibility of developing cardiovascular disease or cancer. Further, tissue-protecting effects of bilirubin were confirmed through animal experiments since it functions to remove different active oxygen and control immunocytes related to inflammation.

Although bilirubin is an industrially useful substance, it has been obtained by extracting from animals and has never been successfully synthesized. When bilirubin is extracted from animals, it is difficult to obtain bilirubin in large quantities while causing high production costs. Further, since bilirubin extracted from animals is a mixture of three regioisomers, it should undergo an additional separation and purification process in order to be utilized as a medicine. It is urgent to develop a method capable of chemically producing bilirubin.

### [Summary of Invention]

### [Problems to be Solved by Invention]

An object of the present invention is to provide a method for synthesizing bilirubin.

### [Means for Solving Problems]

1. A method for synthesizing bilirubin, including preparing a compound represented by Formula 3 below by coupling a compound represented by Formula 1 below with a compound represented by Formula 2 below:

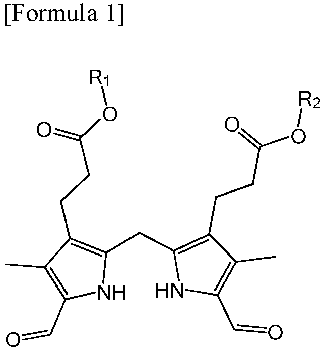

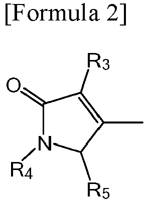

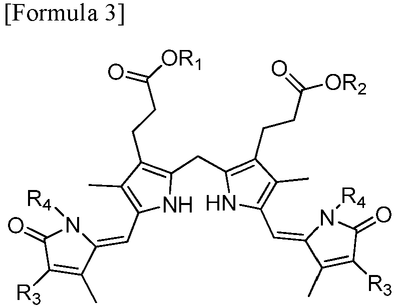

   (wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₃ is hydrogen, a vinyl group, an acetyl group, or an ethyl group substituted with a hydroxyl group, selenide or sulfide; R₄ is hydrogen or a nitrogen protective group; and R₅ is hydrogen, a tosyl or mesyl group).
2. The method according to the above 1, further including reacting the compound represented by Formula 3 with polyethylene glycol (PEG).
3. The method according to the above 1, wherein the compound represented by Formula 1 is reacted with polyethylene glycol (PEG) and then coupled with the compound represented by Formula 2.
4. The method according to the above 1, further including dimerizing a compound represented by Formula 6 below to prepare the compound represented by Formula 1:

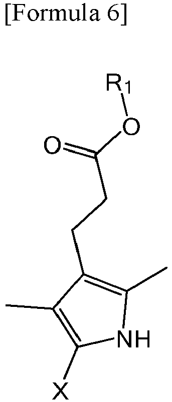

   (wherein, R₁ is the same as R₁ in Formula 1, X is an arylalkyl ester group having 8 to 20 carbon atoms, -CH₂OH, -COOH, halogen atom or hydrogen).
5. The method according to the above 1, further including performing cyclization of a compound represented by Formula 8 below to prepare the compound represented by Formula 2:

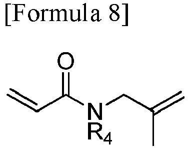

   (wherein, R₄ is the same as R₄ in Formula 2).
6. The method according to the above 1, further including reducing the acetyl group in a compound represented by Formula 11 below to prepare the compound represented by Formula 2: 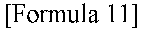 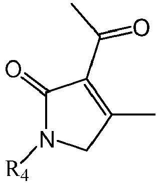 (wherein, R₄ is the same as R₄ in Formula 2).
7. The method according to the above 1, further including dehydrating the hydroxyl group in a compound represented by Formula 12 below to prepare the compound represented by Formula 2:

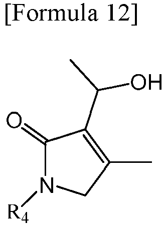

   (wherein, R₄ is the same as R₄ in Formula 2).
8. The method according to the above 1, further including performing cyclization of a compound represented by Formula 13 below to prepare the compound represented by Formula 2:

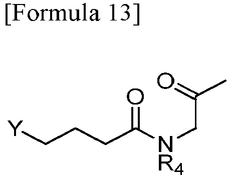

   (wherein, Y is selenide, and R₄ is the same as R₄ in Formula 2).
9. The method according to the above 1, further including oxidizing a compound represented by Formula 14 below to prepare the compound represented by Formula 2: 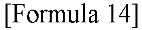 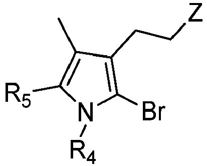 (wherein, Z is sulfide, R₄ and R₅ are the same as R₄ and R₅ in Formula 2).
10. The method according to the above 1, further including preparing bilirubin from the compound represented by Formula 3.
11. The method according to the above 1, wherein the step of preparing a compound is carried out in the presence of a base selected from the group consisting of: piperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidine-3-ol, piperazine, 2,6-dimethylpiperazine, 1-benzyl piperazine, 1-isopropyl piperazine, 2-ethyl piperazine, morpholine, 4-methyl morpholine, 2,6-dimethyl morpholine, ethyl morpholine, azepane, 2-methyl azepan, 4-methyl azepane, 2,2,7,7-tetramethyl azepane, 1,2,2-trimethyl azepane, 1,2-dimethylazepane, 2,7-dimethyl azepane, methyl azepane-4-carboxylate, azocane, 2-methyl azocane, 1,2-dimethyl azocane, 1,2,2-trimethyl azocane, methyl azocane-2-carboxylate, 1-methyl azocane and 2(2-methylphenyl)azocane.
12. The method according to the above 1, wherein the step of preparing a compound is carried out in the presence of a solvent selected from the group consisting of: water, alcohols, ethers, ketones, aliphatic hydrocarbons, aromatic hydrocarbons, halogenated hydrocarbons, alkoxies, nitriles and amides.
13. The method according to the above 1, wherein the step of preparing a compound is carried out at a temperature of -20 to 200 °C.
14. The method according to the above 1, wherein the step of preparing a compound is carried out for 0.5 to 120 hours.
15. The method according to the above 11, wherein the base is added in an amount of 2 to 20 moles based on 1 mole of the compound represented by Formula 1.

### [Advantageous effects]

The method for synthesizing bilirubin of the present invention may be economically performed under mild conditions.

The method for synthesizing bilirubin of the present invention has a high yield and is suitable for mass production.

### [Brief Description of Drawings]

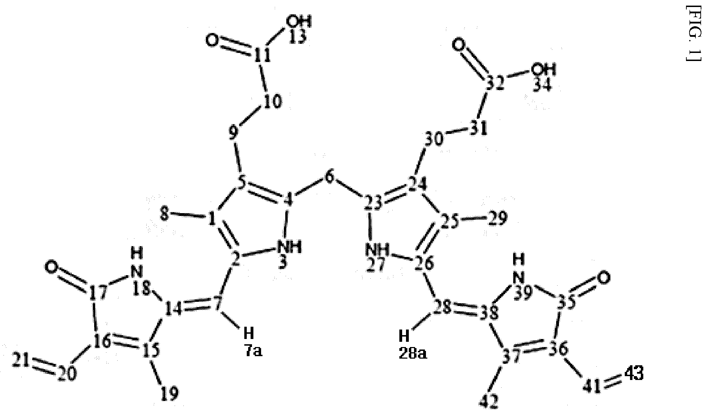

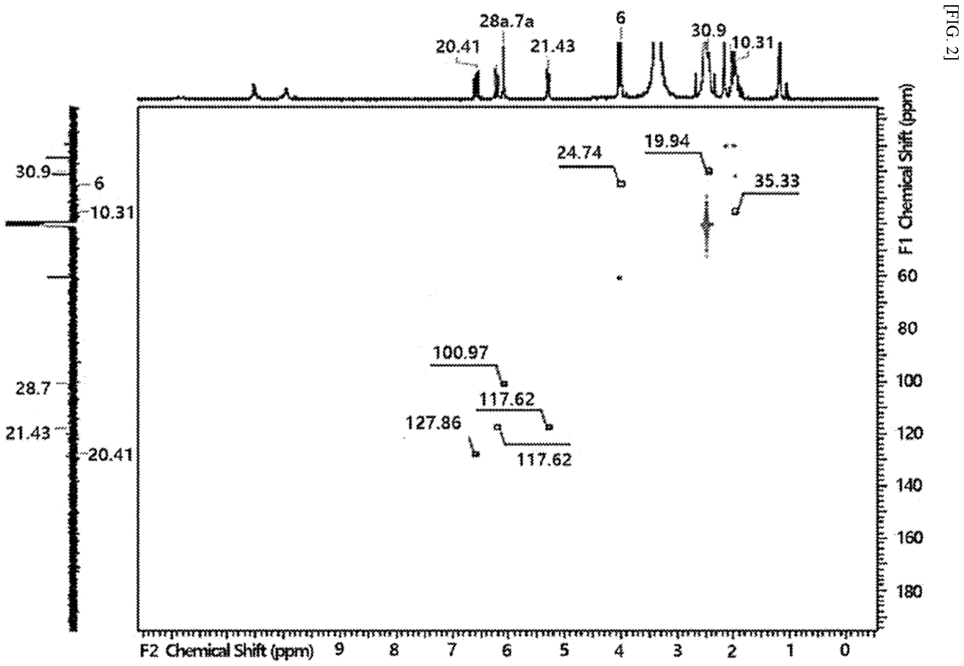

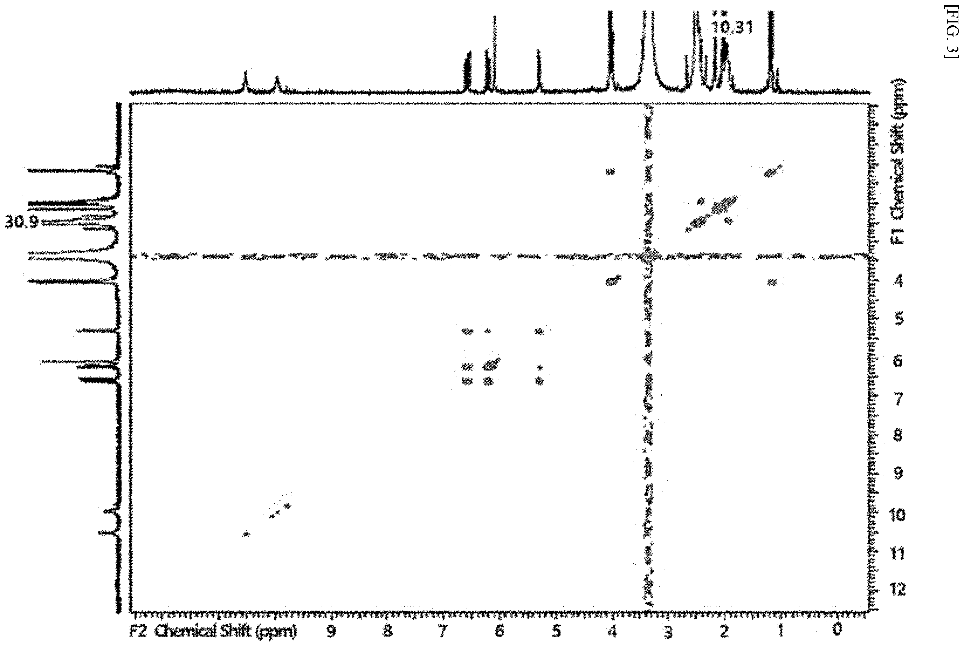

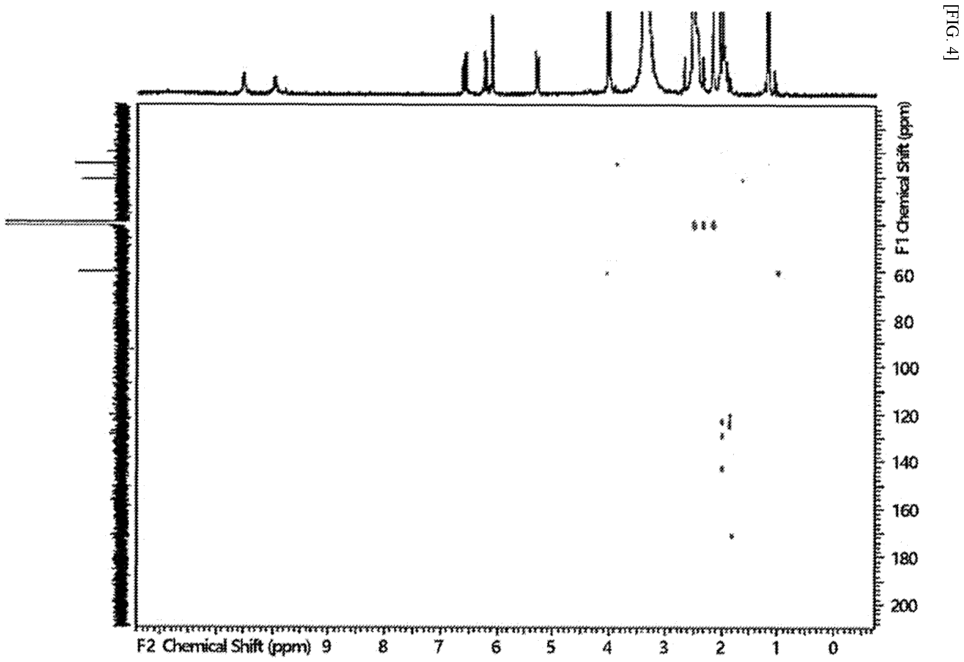

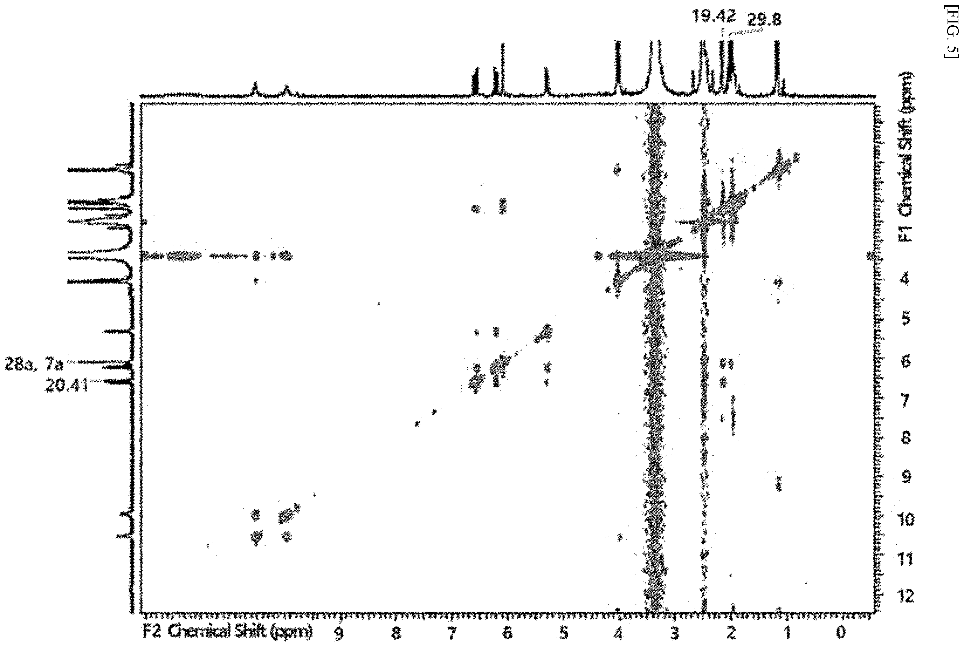

FIGS. 1 to 5 are 2D NMR data of Compound F-3a prepared in Example 29, wherein FIG. 2 is HSQC of F-3a; FIG. 3 is COSY; FIG. 4 is HMBC; and FIG. 5 is NOESY 2D NMR data.

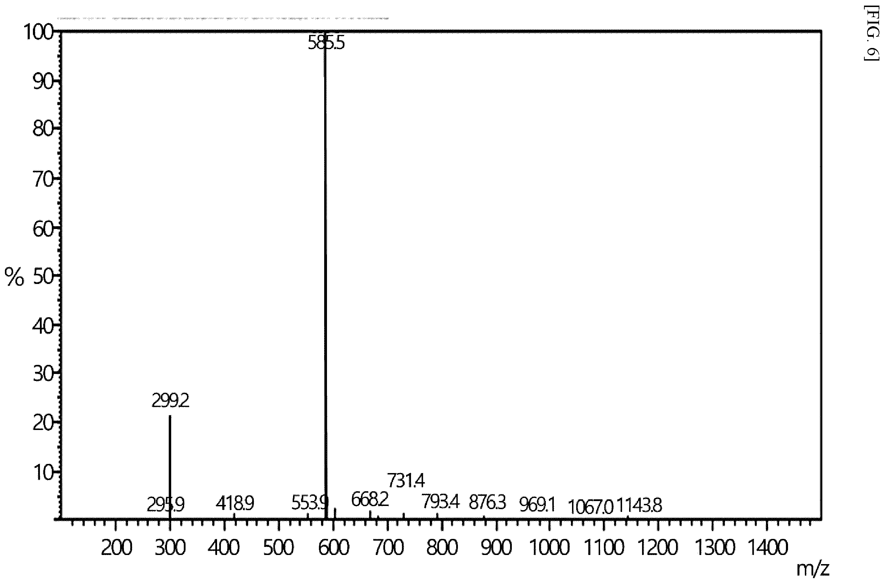

FIG. 6 is Mass data of Compound F-3a prepared in Example 54.

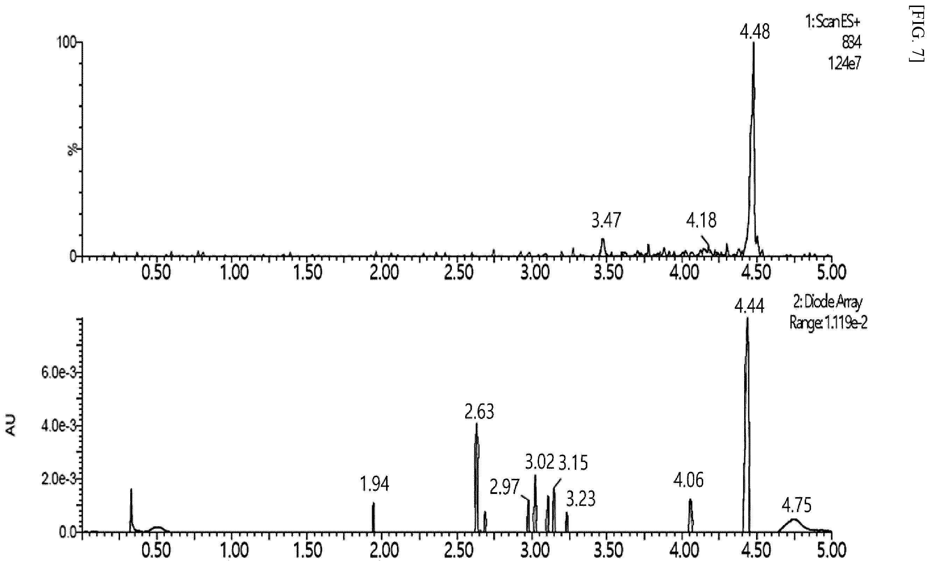

FIG. 7 is LCMS data of Compound D-Ed prepared in Example 59.

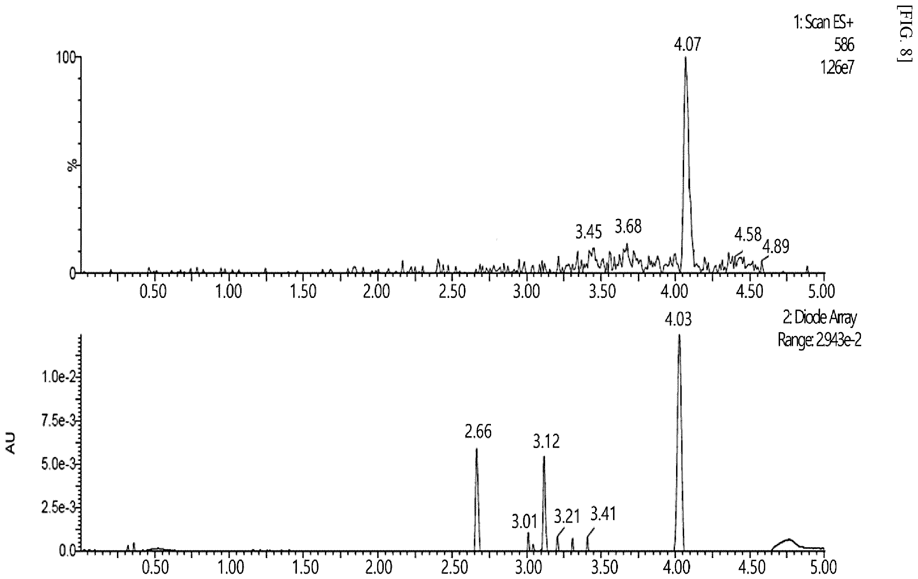

FIG. 8 is LCMS data of Compound F-3a prepared in Example 60.

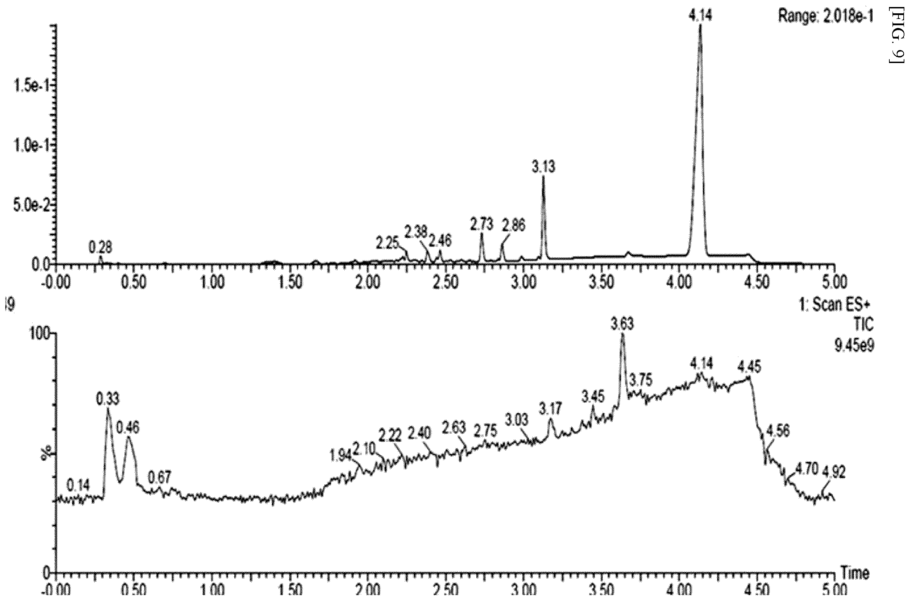

FIG. 9 is LCMS data of Compound F-3a prepared in Example 76.

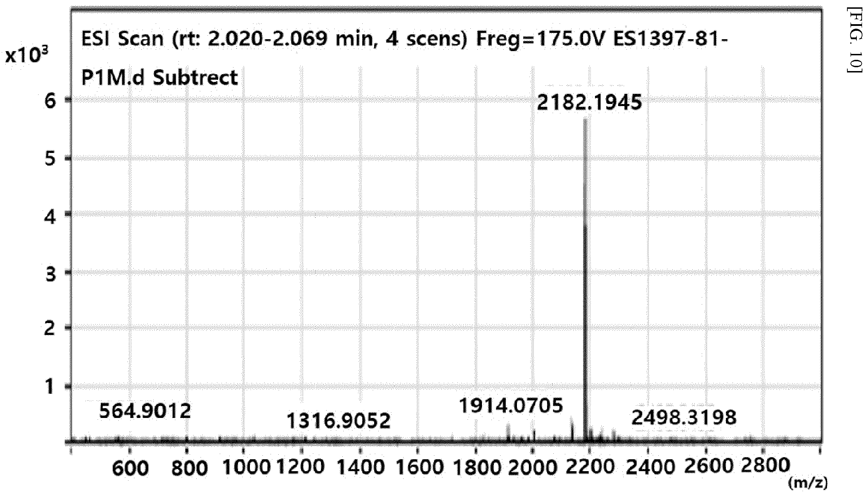

FIG. 10 is Mass data of Compound FP-3a prepared in Example 77.

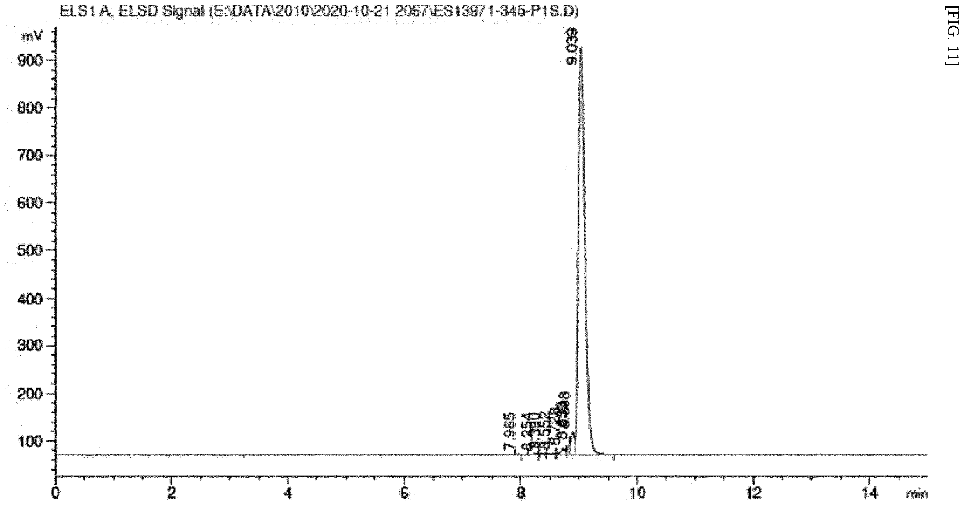

FIG. 11 is HPLC data of Compound FdP-3a prepared in Example 90.

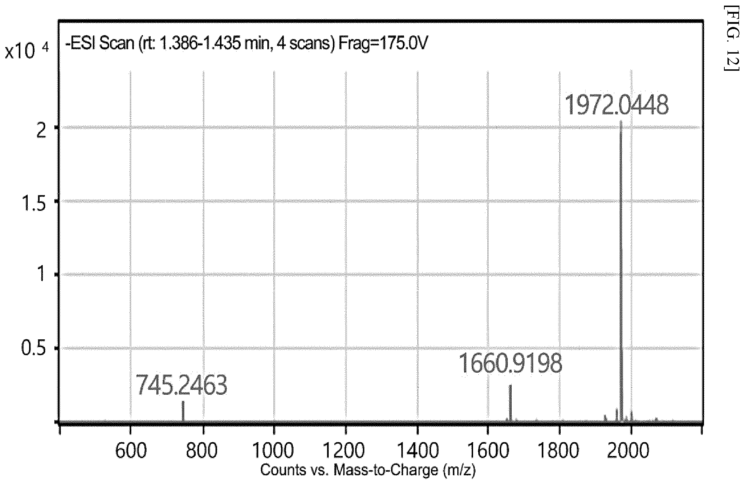

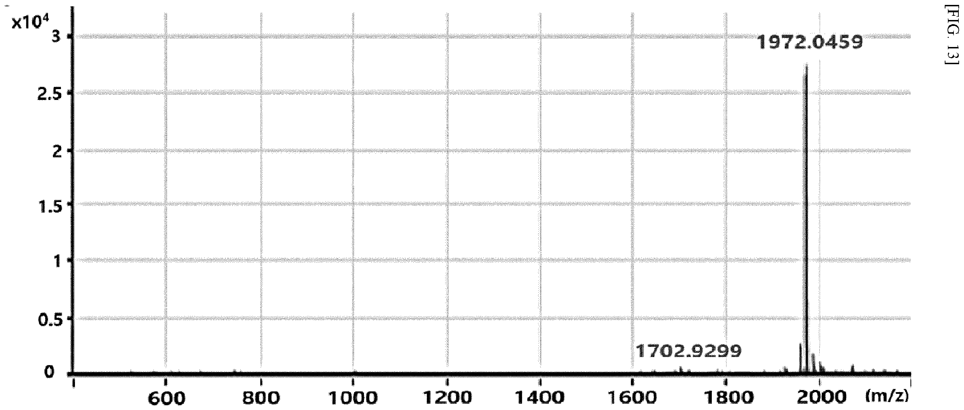

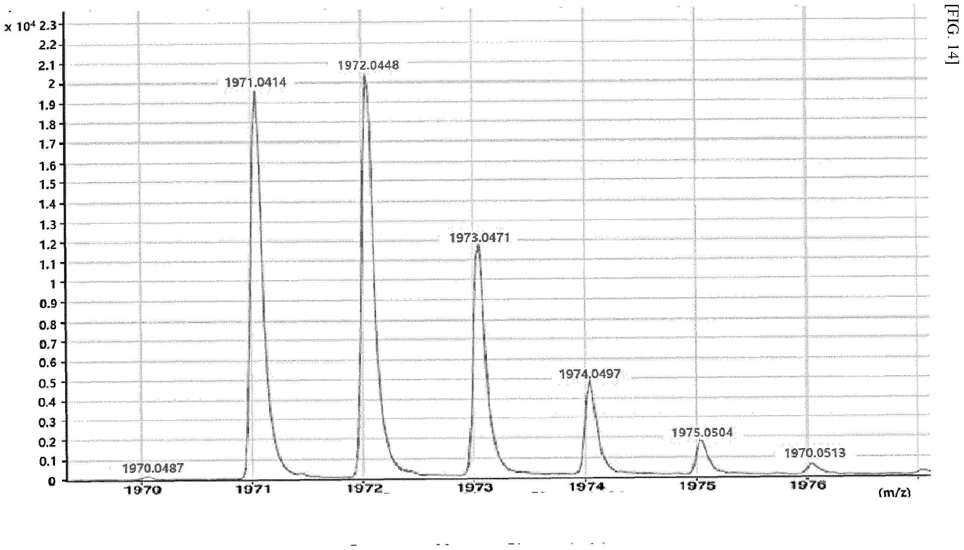

FIGS. 12 to 14 are Mass data of Compound DF prepared in Example 93.

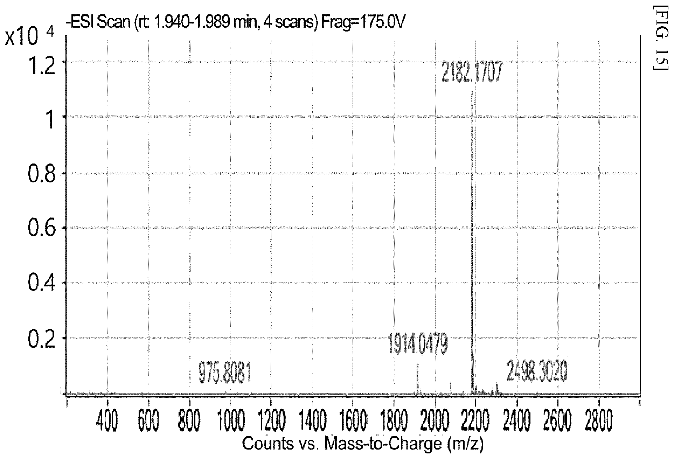

FIG. 15 is Mass data of Compound FP-3a prepared in Example 94.

### [Mode for Carrying out Invention]

The present invention relates to a novel method for synthesizing bilirubin.

As used herein, the term "alkyl" is a straight or branched, substituted or unsubstituted chain hydrocarbon, such as methyl, ethyl, n-propyl, isopropyl, cyclopropyl, n-butyl, sec-butyl, tert-butyl, cyclobutyl, cyclopropylmethyl, n-pentyl, isopentyl, neopentyl, tert-pentyl, cyclopentyl, cyclobutylmethyl, n-hexyl, isohexyl, cyclohexyl, cyclopentylmethyl and the like.

The term "cycloalkyl" is a monocyclic or bicyclic, substituted or unsubstituted cyclic hydrocarbon, such as cyclopropyl, cyclobutyl, cyclopentyl, cyclohexyl, cycloheptyl, cyclooctyl and the like.

The term "heterocycloalkyl" is a monocyclic or bicyclic, substituted or unsubstituted cyclic hydrocarbon containing one or more heteroatoms selected from B, N, O, S, P(=O), Si and P, such as tetrahydropyranyl, azetidyl, 1,4-dioxanyl, piperazinyl, piperidinyl, pyrrolidinyl, morpholinyl, thiomorpholinyl, dihydrofuranyl, dihydroimidazolyl, dihydroindolyl dihydroisooxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrothienyl, dihydrotriazolyl, dihydro-azetidyl, methylenedioxybenzoyl, tetrahydrofuranyl, tetrahydrothienyl and the like.

The term "aryl" is a monocyclic or bicyclic, substituted or unsubstituted aromatic group, such as phenyl, biphenyl, terphenyl, naphthyl, binapthyl, phenylnaphthyl, naphthylphenyl, phenylterphenyl, fluorenyl, phenylfluorenyl, diphenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, spirobifluorenyl, azyurenyl and the like.

"Aryl" includes, for example, phenyl, 1-naphthyl, 2-naphthyl, 1-anthryl, 2-anthryl, 9-anthryl, benzanthryl, 1-phenanthryl, 2-phenanthryl, 3-phenanthryl, 4-phenanthryl, 9-phenanthryl, naphthacetyl, pyrenyl, 1-chrysenyl, 2-chrysenyl, 3-chrysenyl, 4-chrysenyl, 5-chrysenyl, 6-chrysenyl, benzo[c]phenanthryl, benzo[g]chrysenyl, 1-triphenylenyl, 2-triphenylenyl, 3-triphenylenyl, 4-triphenylenyl, 1-fluorenyl, 2-fluorenyl, 3-fluorenyl, 4-fluorenyl, 9-fluorenyl, benzofluorenyl, dibenzofluorenyl, 2-biphenylyl, 3-biphenylyl, 4-biphenylyl, o-terphenyl, m-terphenyl-4-yl, m-terphenyl-3-yl, m-terphenyl-2-yl, p-terphenyl-4-yl, p-terphenyl-3-yl, p-terphenyl-2-yl, m-quaterphenyl, 3-fluoranthenyl, 4-fluoranthenyl, 8-fluoranthenyl, 9-fluoranthenyl, benzofluoranthenyl, o-tolyl, m-tolyl, p-tolyl, 2,3-xylyl, 3,4-xylyl, 2,5-xylyl, mesityl, o-cumenyl, m-cumenyl, p-cumenyl, p-tert-butylphenyl, p-(2-phenylpropyl)phenyl, 4'-methylbiphenylyl, 4"-tert-butyl-p-terphenyl-4-yl, 9,9-dimethyl-1-fluorenyl, 9,9-dimethyl-2-fluorenyl, 9,9-dimethyl-3-fluorenyl, 9,9-dimethyl-4-fluorenyl, 9,9-diphenyl-1-fluorenyl, 9,9-diphenyl-2-fluorenyl, 9,9-diphenyl-3-fluorenyl, 9,9-diphenyl-4-fluorenyl and the like.

The term "heteroaryl" refers to a monocyclic or bicyclic, substituted or unsubstituted aromatic group containing one or more heteroatoms selected from B, N, O, S, P(=O), Si and P, such as benzothienyl, benzoxazolyl, benzofuranyl, benzimidazolyl, benzthiazolyl, benzotriazolyl, sinnolinyl, furyl, imidazolyl, tetrazolyl, indazolyl, indolyl, isoxazolyl, isoquinolinyl, isothiazolyl, naphthyridinyl, oxadiazolyl, oxazolyl, isoxazolyl, purinyl, thiazolyl, isothiazolyl, thienopyridinyl, thienyl, thiadiazolyl, pyridinyl, pyridazinyl, pyrimidinyl, pyrazinyl, pyrazolyl, pyrrolyl, pyrido[2,3-d]pyrimidinyl, pyrrolo[2,3-b]pyridinyl, quinazolinyl, quinolinyl, thieno[2,3-c]pyridinyl, triazinyl and the like.

The term "arylalkyl" refers to an alkyl group in which at least one of the substituents is substituted with aryl, while "aryl" and "alkyl" are as defined above. For example, this includes benzyl, phenylethyl, phenylpropyl, phenylbutyl, phenylhexyl, naphthylethyl, naphthylpropyl, naphthylbutyl, naphthylhexyl, anthracenylmethyl, anthracenylethyl, anthracenylpropyl, anthracenylbutyl, phenanthrylmethyl, phenanthryl ethyl, phenanthrylpropyl, triphenylmethyl, triphenylethyl, triphenylpropyl, pyrenylmethyl, pyrenylethyl, pyrenylpropyl, phenylanthracenemethyl, phenylanthraceneethyl, phenylanthracenepropyl, perylenylmethyl, perylenylethyl, perylenylpropyl, chrysenylmethyl, chrysenylethyl, chrysenylpropyl, fluorenylmethyl, fluorenylethyl, fluorenylpropyl and the like.

The term "heteroarylalkyl" refers to an alkyl group in which at least one of the substituents is substituted with heteroaryl, while heteroaryl and alkyl are as defined above. For example, this includes pyridinylmethyl, pyridinylethyl, pyridinylpropyl, pyridinylbutyl, pyrimidinylmethyl, pyrimidinylethyl, pyrimidinylpropyl, pyrazolylmethyl, pyrazolylethyl, pyrazolylmethyl, pyrazolylethyl, pyrazolylpropyl, quinolinylmethyl, quinolinylethyl, quinolinylpropyl and the like.

The term "substituted" refers to inclusion of at least one substituent, for example, one or two or more of halogen atom, nitro, hydroxyl, cyano, amino, thiol, carboxyl, amide, nitrile, sulfide, disulfide, sulfenyl, formyl, formyloxy, formylamino, aryl or substituted aryl.

In the case where no substituent is described in a formula of the present invention even though it is a site requiring a substituent, it is considered that a hydrogen substituent is omitted.

The present invention relates to a method for synthesizing bilirubin, which includes preparing a compound represented by Formula 3 below by coupling a compound represented by Formula 1 below with a compound represented by Formula 2 below.

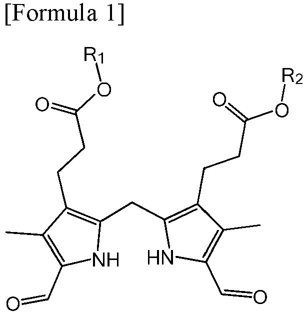

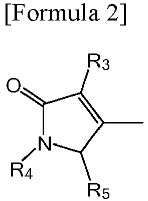

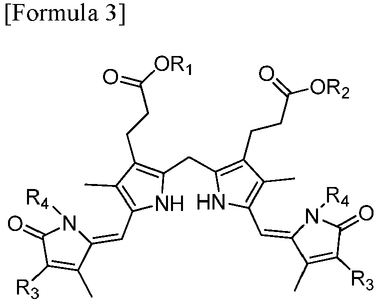

Wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms.

The number of carbon atoms in R₁ and R₂ may be appropriately selected within a range that does not affect a coupling reaction between the compound represented by Formula 1 and the compound represented by Formula 2.

For example, R₁ and R₂ may be each independently an alkyl group having 1 to 10 carbon atoms, an aryl group having 6 to 10 carbon atoms, a heteroaryl group having 2 to 10 carbon atoms, an arylalkyl group having 7 to 10 carbon atoms, or a heteroarylalkyl group having 3 to 10 carbon atoms.

Further, R₁ and R₂ may be each independently selected from an alkyl group having 1 to 5 carbon atoms, an aryl group having 6 to 10 carbon atoms, a heteroaryl group having 4 to 10 carbon atoms, an arylalkyl group having 7 to 10 carbon atoms, or a heteroarylalkyl group having 5 to 10 carbon atoms.

R₃ may be hydrogen, a vinyl or acetyl group; or an ethyl group substituted with a hydroxyl group, selenide or sulfide.

Herein, selenide is a functional group having a structure of Formula 4 below, while sulfide is a functional group having a structure of Formula 5 below.

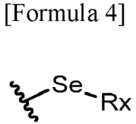

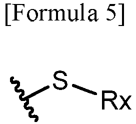

Wherein, in Formulas 4 and 5, Rx may be hydrogen, or a substituted or unsubstituted, straight-chain or branched alkyl, cycloalkyl, heterocycloalkyl, aryl, heteroaryl, arylalkyl, or heteroarylalkyl group.

For example, Rx is an alkyl group having 1 to 12 carbon atoms, a cycloalkyl group having 5 to 20 carbon atoms, a heterocycloalkyl group having 2 to 20 carbon atoms, an aryl group having 5 to 20 carbon atoms, a heteroaryl group having 2 to 20 carbon atoms, an arylalkyl group having 6 to 20 carbon atoms, or a heterocycloalkyl group having 3 to 20 carbon atoms.

For example, Rx is a phenyl or p-tolyl group.

R₃ may be an ethyl group substituted with a hydroxyl group, for example, may be a functional group in which a hydroxyl group is substituted at the position of carbon No. 1 of an ethyl group.

R₃ may be an ethyl group substituted with selenide, for example, may be a functional group in which selenide is substituted at the position of carbon No. 2 of an ethyl group.

R₃ may be an ethyl group substituted with sulfide, for example, may be a functional group in which sulfide is substituted at the position of carbon No. 2 of an ethyl group.

R₄ is hydrogen or a nitrogen protecting group.

Herein, the nitrogen-protecting group is not limited to a specific one as long as it is a substituent capable of protecting the nitrogen atom to which R₄ is bonded, and for example, may be selected from the group consisting of -COORₓ (Rₓ is as defined above), tert-butyloxycarbonyl (Boc), trityl (-CPh₃), tosyl group (SOOPhCH₃), 9-fluorenylmethyloxy carbonyl (Fmoc), carboxybenzyl group (Cbz), p-methoxybenzyl carbonyl (Moz), acetyl (Ac), benzoyl (Bz), p-methoxybenzyl (PMB), 3,4-dimethoxybenzyl (DMPM), p-methoxyphenyl (PMP), 2-naphthylmethyl ether (Nap), and trichloroethyl chloroformate (Troc).

R₅ is hydrogen, a tosyl ( 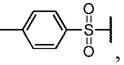 Ts or Tos) or methyl group ( 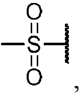 Ms).

When the nitrogen-protecting group of R₄ of Formula 2 remains after the compound represented by Formula 1 and the compound represented by Formula 2 are coupled, a step of removing the nitrogen-protecting group may be additionally required.

The compound represented by Formula 1 and the compound represented by Formula 2 may be bonded in a molar ratio of 1:2. The compound represented by Formula 1 and the compound represented by Formula 2 may be introduced at a molar ratio of 1: 2 to 10, 1: 2 to 5, 1: 2 to 4, or 1: 2 to 3 depending on the reaction.

The coupling reaction is carried out in the presence of a solvent and base.

The solvent is an inorganic solvent or an organic solvent. The organic solvent may be, for example, alcohols, ethers, ketones, aliphatic hydrocarbons, aromatic hydrocarbons, halogenated hydrocarbons, alkoxies, nitriles or amides. Solvents belonging to these classes are, for example, listed in Table 1. The inorganic solvent is, for example, water.

**[TABLE 1]**

| | |
|---|---|
| Division | Organic solvent |
| Alcohols | Methanol, ethanol, propanol, isopropanol, ethylene glycol |
| Ethers | Diethylether, tetrahydrofuran (THF), 2-methyl tetrahydrofuran, dioxanes |
| Ketones | Methyl cellosolve, ethyl cellosolve, butyl cellosolve, methylethyl ketone, acetone |
| Aliphatic hydrocarbons | Hexane, heptane, octane |
| Aromatic hydrocarbons | Benzene, toluene, xylene |
| Halogenated hydrocarbons | Dichloromethane (DCM), chloroform, chlorobenzene |
| Alkoxies | Methoxyethane, dimethoxyethane (DME), methoxypropane, dimethoxypropane |
| Nitriles | Acetonitrile, benzonitrile, trinitrile |

The base is an organic base or an inorganic base. The base is preferably a stronger base than the compound represented by Formula 2.

As the organic base, it is preferable to use an amine organic base. For example, it may be chain type amine organic bases such as methylamine, ethylamine, dimethylamine, diethylamine, ethylmethylamine, propylamine, dipropylamine, methylpropylamine, ethylpropylamine, diisopropylamine, N-methylcyclohexylamine or trimethylamine, etc.; or cyclic amine organic bases such as aziridine, azetidine, oxaziridine, azetidine, diazetidine, imidazolidine, pyrazolidine, oxazolidine, isoxazolidine, thiazolidine, isothiazolidine, piperidine, 2-methylpiperidine, 2-ethylpiperidine, 2,6-dimethylpiperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidin-3-ol, piperazine, 2,6-dimethylpiperazine, 1-benzylpiperazine, 1-isopropylpiperazine, 2-ethylpiperazine, N-propylpiperazine, morpholine, thiomorpholine, 4-methyl morpholine, 2,6-dimethyl morpholine, ethyl morpholine, azepane, 2-methyl azepane, 4-methyl azepane, 2,2,7,7-tetramethyl azepane, 1,2,2-trimethyl azepane, 1,2-dimethyl azepane, 2,7-dimethyl azepane, azocane, 1,2-dimethyl azocane, 1,2,2-trimethyl azocane, methyl azocane-2-carboxylate, 1-methyl azocane, 2-(2-methylphenyl) azocane or proline, etc.

The organic base is preferably piperidine, pyrrolidine, morpholine, piperazine, azepane, azocane, N-methylpiperidine, N-ethylpiperidine or proline.

The inorganic base may be, for example, LiOH, KOH or NaOH.

The base may be included in an amount of 2 to 20 moles, 2 to 15 moles, 2 to 10 moles, 4 to 20 moles, 4 to 15 moles, 4 to 10 moles, or 5 to 20 moles, 5 to 15 moles, 5 to 10 moles, 6 to 20 moles, 6 to 15 moles or 6 to 10 moles based on 1 mole of the compound represented by Formula 1.

The coupling reaction temperature of the present invention is -20 to 200 °C. For example, it may be 30 to 180 °C, 30 to 150 °C, 30 to 120 °C, 30 to 100 °C, 40 to 150 °C, 40 to 140 °C, 40 to 120 °C, 40 to 100 °C, 50 to 150 °C, 50 to 120 °C, or 50 to 100 °C. The optimum reaction temperature may vary depending on the solvent and base used.

The coupling reaction time of the present invention may be 10 minutes to 120 hours, for example, 1 to 72 hours, 1 to 48 hours, 1 to 24 hours, 3 to 72 hours, 3 to 48 hours, 3 to 24 hours, 6 to 72 hours, 6 to 48 hours, or 6 to 24 hours. The optimal reaction time may vary depending on the solvent and base used.

The method for synthesizing bilirubin of the present invention may further include converting R₁ and/or R₂ of the compound represented by Formula 3 into hydrogen through a saponification reaction. For example, when R₁ and R₂ of the compound represented by Formula 3 are a methyl group, a base such as LiOH, KOH or NaOH may be added to the compound represented by Formula 3 in order to replace the methyl group with hydrogen.

The solvent used for the saponification reaction is not particularly limited. As the solvent for saponification, the same solvent as for the coupling reaction may be used. For example, it may be methanol, ethanol, 2-propanol, tetrahydrofuran (THF), 2-methyltetrahydrofuran (2-MeTHF), 1,4-dioxane, acetonitrile, N,N-dimethylformamide (DMF), t-butanol, dimethoxyethane (DME), dichloromethane (DCM), isopropyl alcohol or the like.

The saponification may be carried out under conditions known in the art. For example, it may be performed at 10 to 150 °C for 1 to 72 hours, or at 10 to 60 °C for 1 to 48 hours.

The method for synthesizing bilirubin of the present invention may further include a PEGylation step of reacting the compound represented by Formula 3 with polyethylene glycol (PEG).

The method for synthesizing bilirubin of the present invention may include a step of PEGylating the compound represented by Formula 1 with polyethylene glycol (PEG) and then coupling the resulting product with the compound represented by Formula 2.

PEGylated bilirubin has improved water solubility.

Polyethylene glycol is, for example, mPEGₙ -NH₂ (methoxy polyethylene glycol-amine, n=5 to 60). Herein, n is the number of -CH₂-CH₂-O- repeating units of methoxy polyethylene glycol-amine, which may range from 5 to 60, 10 to 50, 10 to 40, 20 to 40, 10 to 30, or 20 to 30.

PEGylation includes mono-PEGylation in which either O-R₁ or O-R₂ is PEGylated, and bi-PEGylation in which both of them are PEGylated.

In the PEGylation reaction, polyethylene glycol may be added in an appropriate amount in consideration of the number of moles of the compound represented by Formula 1 or Formula 3. For example, polyethylene glycol may be added in an amount of 0.1 to 10 moles, 0.1 to 8 moles, 0.1 to 5 moles, 0.3 to 8 moles, 0.3 to 5 moles, or 0.3 to 4 moles, or 0.3 to 3 moles based on 1 mole of the compound represented by Formula 1 or Formula 3.

As reagents for PEGylation reaction, CDI (1,1-carbonyldiimidazole), CMPI (2-chloro-1-methylpyridinium iodide), BEP (2-bromo-1-ethyl-pyridinium tetrafluoroborate), EDCI (1-ethyl-3-(3-dimethylaminopropyl)carbodiimide), HATU (1-[bis(dimethylamino)methylene]-1H-1,2,3-triazolo[4,5-b]pyridinium 3-oxide hexafluorophosphate; hexafluorophosphate azabenzotriazole tetramethyl uronium, DCC (N,N'-dicyclohexylcarbodiimide) or HOBt (hydroxybenzotriazole), etc. may be used, but it is not limited thereto.

The reagent for PEGylation reaction may be added in an amount of 0.3 to 5 moles, 0.3 to 3 moles, 0.5 to 5 moles, 0.5 to 3 moles, 0.5 to 2.5 moles or 0.5 to 2 moles based on 1 mole of the compound represented by Formula 1 or Formula 3, but it is not limited thereto.

The solvent for PEGylation reaction is not particularly limited. As the solvent for PEGylation reaction, the same solvent as for coupling reaction may be used. For example, it may be DMSO (dimethyl sulfoxide), DMF (dimethylformamide), DMA (dimethylacetamide) or pyridine.

The PEGylation reaction may be carried out in the presence of a base. The base may be selected within the range previously exemplified as the bases in the coupling reaction, and is preferably DIPEA (N,N-Diisopropylethylamine) or pyridine.

The PEGylation reaction may be carried out at 10 to 100 °C, such as 10 to 80 °C, 20 to 60 °C, 20 to 50 °C, or 20 to 30 °C.

The PEGylation reaction may be carried out for 1 to 24 hours, 1 to 18 hours, and 1 to 12 hours, but it is not limited thereto.

In one embodiment, the PEGylation reaction may be performed by adding 0.3 to 5 moles of polyethylene glycol and 0.5 to 5 moles of a coupling reagent (CDI, EDCI, CMPI, etc.) based on 1 mole of the compound represented by Formula 1 or Formula 3, and this reaction may be carried out at 20 to 40 °C for 0.5 to 24 hours.

The compound represented by Formula 1, as well as the compound represented by Formula 2 as reactants in the bilirubin synthesis method of the present invention may be prepared as follows.

The compound represented by Formula 1 may be prepared by dimerizing a compound represented by Formula 6 below and replacing X of the product with -C(=O)H.

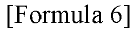

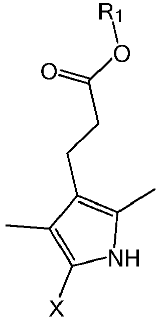

Wherein R₁ in Formula 6 is the same as R₁ in Formula 1, and X is an arylalkyl ester group having 8 to 20 carbon atoms, -CH₂OH, -COOH, a halogen atom, or hydrogen.

The arylalkyl ester group is a functional group in which R_{Y} in Formula 7 below is an arylalkyl group.

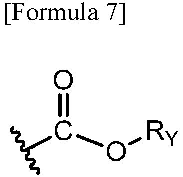

The arylalkyl group of Formula 7 is the same as the arylalkyl group of Formula 1.

The number of carbon atoms in the arylalkyl ester group may be appropriately selected within a range that does not affect the dimerization of the compound represented by Formula 6. For example, it may have 8 to 20 carbon atoms, 8 to 18 carbon atoms, 8 to 15 carbon atoms, or 8 to 12 carbon atoms.

A method for substituting X of the product with an aldehyde group is, for example, as follows (1) to (5).
(1) When X is an arylalkyl ester group, the arylalkyl ester group is reduced to -COOH by hydrogenation, then -COOH is reduced or removed. Thereafter, -C(=O)H may be added to replace X with an aldehyde group.
   In one embodiment, the hydrogenation may be carried out under a Pd/C catalyst. For example, the arylalkyl ester group is -C(=O)OBn (Bn=benzyl), then -C(=O)OBn is reduced to - COOH by a hydrogenation reaction under a Pd/C catalyst. Thereafter, -COOH is removed to replace the arylalkyl ester group with -C(=O)H.
(2) When X is -CH₂OH, X is oxidized by a known method and replaced with -C(=O)H.
(3) When X is -COOH, -COOH is reduced or removed, followed by adding -C(=O)H to replace X with an aldehyde group.
(4) When X is a halogen atom, the halogen atom is substituted with an aldehyde group by a carbonylation reaction. A known method can be used for the carbonylation reaction. For example, the carbonylation reaction may be carried out using carbon monoxide and palladium.
(5) When X is hydrogen, hydrogen is replaced with an aldehyde group by an aldehyde addition reaction. A known method may be used for the aldehyde addition reaction. For example, an aldehyde addition reaction may be performed using BuLi and DMF.

The dimerization of the compound represented by Formula 6 may be carried out under, for example, bromine (Br₂) conditions. A solvent for dimerization is not particularly limited. For example, as the organic solvent, the solvents in Table 1 exemplified as the coupling reaction solvents may be used.

The dimerization reaction may be performed at 10 to 100 °C, for example, 10 to 80 °C, 20 to 60 °C, 20 to 50 °C, or 20 to 30 °C.

The dimerization reaction may be performed for 1 to 24 hours, 1 to 18 hours, or 1 to 12 hours, but it is not limited thereto.

The compound represented by Formula 2 (R₃ = hydrogen) may be prepared by cyclizing the compound represented by Formula 8.

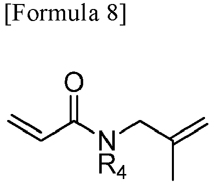

Wherein R₄ is the same as R₄ in Formula 2.

The cyclization reaction of the compound represented by Formula 8 may be performed under a Grubbs catalyst. When the compound represented by Formula 8 is cyclized, a compound represented by Formula 2 may be produced.

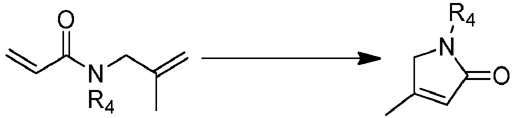

The compound represented by Formula 2 (R₃ = acetyl) may be prepared by reacting a compound represented by Formula 9 with a compound represented by Formula 10 below. 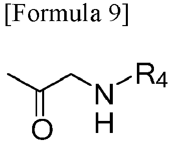 (wherein, R₄ is the same as R₄ in Formula 2)

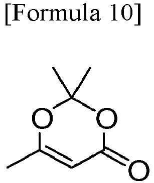

The compound represented by Formula 2 (R₃ = an ethyl group substituted with a hydroxyl group) may be prepared by reducing the acetyl group of a compound represented by Formula 11 below. 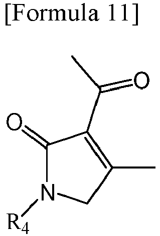 (wherein, R₄ is the same as R₄ in Formula 2).

The reaction of reducing the acetyl group may be performed by a known method, and the conditions for the same may be implemented within a range of solvent, temperature, time, and the like, in the coupling reaction. For example, the acetyl group may be reduced using MeOH as a solvent and NaBH₄ and CeCl₃·7H₂O as a reduction reaction reagent, or THF as a solvent and DIBAL as a reduction reaction reagent.

The compound represented by Formula 2 (R₃ = a vinyl group) may be prepared by dehydrating the hydroxyl group of a compound represented by Formula 12 below.

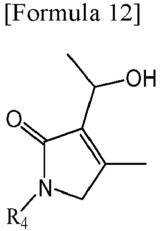

Wherein R₄ is the same as R₄ in Formula 2.

The reaction of dehydrating the hydroxyl group may be performed by a known method, and the conditions may be performed within a range of the solvent, temperature, time, and the like in the previous coupling reaction. For example, the hydroxyl group may be dehydrated using DCM (dichloromethane) as a solvent and POCl₃ and TEA as reaction reagents.

The compound represented by Formula 2 (R₃ = an ethyl group substituted with selenide) may be prepared by cyclizing a compound represented by Formula 13.

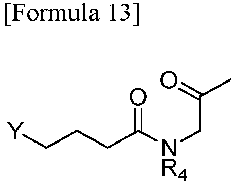

Wherein Y is selenide, and R₄ is the same as R₄ in Formula 2.

The cyclization reaction of the compound represented by Formula 13 may be performed within a range of the solvent, temperature, time, etc. for the coupling reaction. When the compound represented by Formula 13 is cyclized, the compound represented by Formula 2 may be produced.

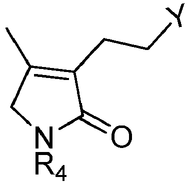

The compound represented by Formula 13 may be prepared, for example, as follows.

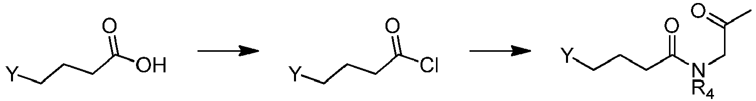

The compound represented by Formula 2 (R₃ = an ethyl group substituted with sulfide) may be prepared by oxidizing a compound represented by Formula 14 below.

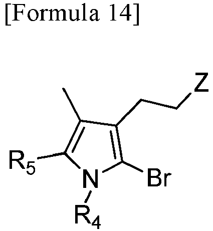

Wherein Z is sulfide, and R₄ and R₅ are the same as R₄ and R₅ in Formula 2.

The compound represented by Formula 2 obtained by oxidizing the compound represented by Formula 14 (R₃ = an ethyl group substituted with sulfide) is as follows.

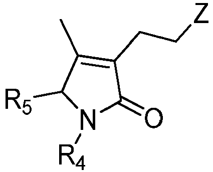

The oxidation reaction of the compound represented by Formula 14 may be performed by treating with NaI after treating with mCPBA. The oxidation reaction of the compound represented by Formula 14 may be carried out within a range of the solvent, temperature, time, etc. for the coupling reaction, but it is not limited thereto.

The compound represented by Formula 14 may be prepared as follows.

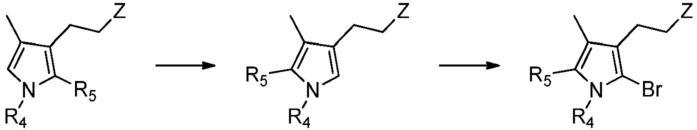

When R₃ of the compound represented by Formula 3 of the present invention is hydrogen or an acetyl group; or, an ethyl group substituted with a hydroxyl group, selenide or sulfide, a step of converting these substituents to a vinyl group should be additionally performed.
(1) When R₃ is hydrogen, conversion to a vinyl group may be carried out by bromination and carbon-carbon coupling reaction. Bromination may be carried out by a known method, and for example, Br₂ may be used as a reagent for bromination reaction.

The carbon-carbon coupling reaction may be, for example, Stille reaction, Suzuki-Miyaura reaction, Heck reaction, or Grignard reaction.

The carbon-carbon coupling reaction may be carried out, for example, as follows.

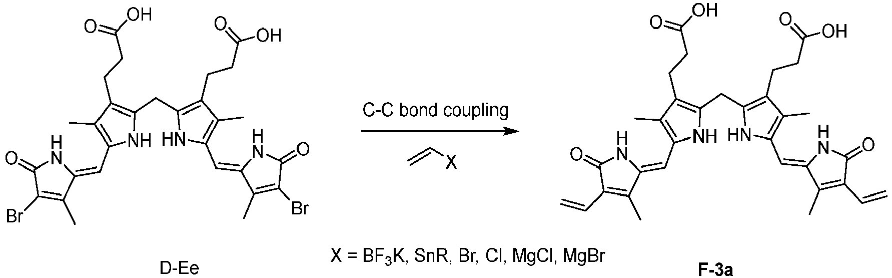

The reaction reagent for carbon-carbon coupling may be, for example, BF₃K, SnR (R is C₁ -C₂₀ alkyl), Br, Cl, MgCl or MgBr, but it is not limited thereto.

Stille reaction may be performed by a known method, and may be, for example, a reaction with a compound represented by Formula 15 below, but it is not limited thereto.

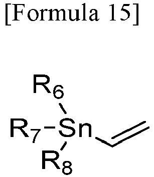

Wherein R₆ to R₈ are each independently an alkyl group having 1 to 20 carbon atoms, 1 to 15 carbon atoms, or 1 to 10 carbon atoms.

Suzuki reaction may be performed by a known method, and may be, for example, a reaction with a compound represented by Formula 16 below, but it is not limited thereto.

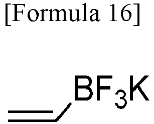

(2) When R₃ is an acetyl group, conversion to a vinyl group may be carried out by reduction and dehydration of the acetyl group. Reduction and dehydration of acetyl groups may be performed by known methods.

(3) When R₃ is an ethyl group substituted with a hydroxyl group, conversion to a vinyl group may be carried out by a dehydration reaction of the hydroxyl group. The dehydration reaction of the hydroxyl group may be performed by a known method.

(4) When R₃ is an ethyl group substituted with selenide, conversion to a vinyl group may be carried out by an oxidation reaction of selenide. For example, the compound of Formula 3 may be converted into a vinyl group by oxidation in the presence of HOAc and H₂O₂ as follows.

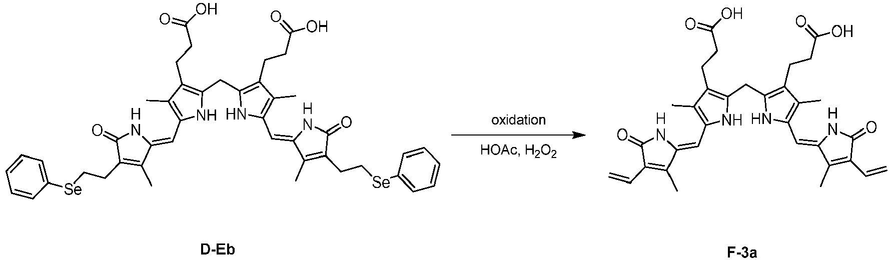

(5) When R₃ is an ethyl group substituted with sulfide, conversion to a vinyl group may be carried out by an oxidation reaction of sulfide. For example, the compound of Formula 3 may be oxidized with mCPBA and then deprotected with pyridine as follows.

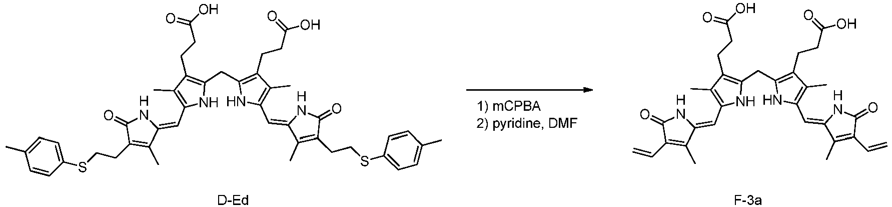

Hereinafter, the present invention will be described in more detail through examples.

### <EXAMPLE>

### 1. Preparation of the compound represented by Formula 1

Compound C, Compound D, Compound C1 and Compound C2 corresponding to the compound represented by Formula 1 herein were prepared as follows.

### Example 1: Preparation of Compound C

### (1-1) Preparation of Compound A

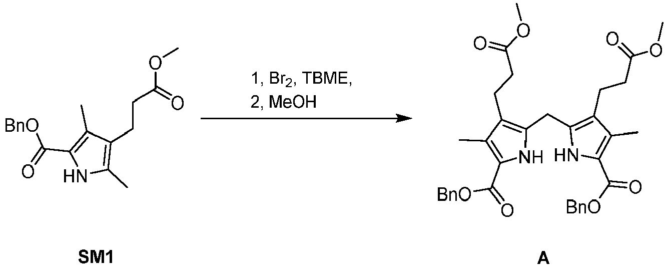

A mixture of Br₂ (53.2 g, 333 mmol, 1.4 equiv.) in MTBE (375 mL) was added dropwise to a mixture of compound SM1 (75.0 g, 238 mmol, 1.0 equiv.) in MTBE (1125 mL) at 20 °C under nitrogen condition. The mixture was stirred at 20 °C for 1 hour, followed by confirming complete reaction through TLC. The solvent was removed under reduced pressure condition. Then, methanol (546 mL) was added to the mixture. The mixture was stirred at 50 °C for 12 hours, followed by confirming complete consumption of the reactants through TLC. The mixture was cooled to 20 °C and concentrated under reduced pressure condition. After the mixture was triturated with methanol (100 mL) at 20 °C, the filtered product was washed with methanol (50 mL x 2) to yield Compound A as a gray solid (59.0 g, 95.9 mmol, yield: 81%).

¹H NMR (400 MHz, CDCl₃) δ 9.11 (s, 2H), 7.41 - 7.25 (m, 10H), 5.26 (s, 4H), 3.97 (s, 2H), 3.58 (s, 6H), 2.77 (t, *J=* 7.2 Hz, 4H), 2.52 (t, *J=* 6.8 Hz, 4H), 2.29 (s, 6H).

### (1-2) Preparation of Compound B

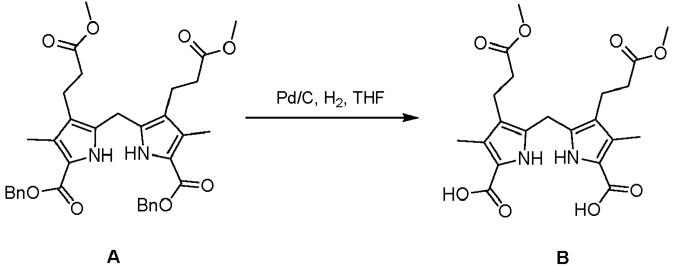

To a mixture of Compound A (50.0 g, 81.3 mmol, 1.0 equiv.) prepared above in THF (650 mL), Pd/C (5.00 g, 10 mol%) was added under nitrogen condition. The mixture was degassed under vacuum condition and filled with H₂ several times. The mixture was stirred at 20 °C for 16 hours under H₂ condition (15 psi). A mixture of Na₂CO₃ (8.62g, 81.3 mmol) and H₂O (50 mL) was added to the above mixture and stirred for 0.5 hours. The mixture was filtered and the filtrate was adjusted to pH 7 by adding acetic acid (ca. 10 mL) thereto. The precipitate was filtered and dried to yield Compound B as a pink solid (34.0 g, 78.3 mmol, yield: 96%).

¹H NMR (400 MHz, DMSO-*d₆*) δ 11.09 (s, 2H), 3.78 (s, 2H), 3.56 (s, 6H), 2.56 (t, *J* = 7.2 Hz, 4H), 2.16 - 2.10 (m, 10H).

### (1-3) Preparation of Compound C

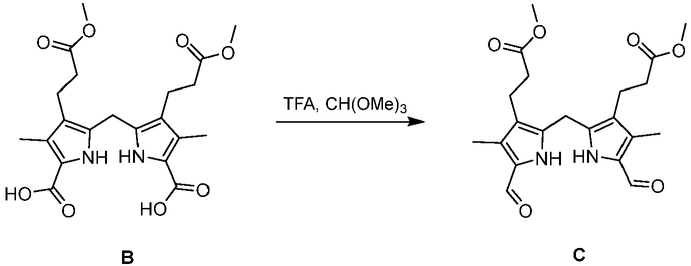

Compound B (20.0 g, 46.1 mmol, 1.0 equiv.) prepared above was added to TFA (190 mL) at 0 °C. The mixture was stirred at 0 °C for 1 hour under nitrogen condition, and trimethoxymethane (55.2 g, 520 mmol, 11.3 equiv.) was added to the mixture at 0 °C. The mixture was then stirred at 0 °C for 1 hour and monitored by LCMS. The mixture was added to 1.7 L of water and stirred for 10 minutes. The resulting precipitate was filtered and washed with 0.3 L of water. The filtered solid was triturated with ethanol (0.2 L) and ammonium hydroxide (0.4 L) at 20 °C for 30 minutes. The precipitate was filtered to give a yellow powder then washed with water (0.3 L). Methanol (0.4 L) was added to the product and refluxed for 10 minutes. After cooling the mixture to room temperature, the precipitate was filtered and washed with cold methanol (0.1 L) to yield Compound C corresponding to the compound represented by Formula 1 herein as a brown solid (12.0 g, 29.8 mmol, yield: 65%).

¹H NMR (400 MHz, CDCl₃) δ 10.19 - 10.00 (m, 2H), 9.47 (s, 2H), 4.05 (s, 2H), 3.71 (s, 6H), 2.80 (t, *J* = 7.2 Hz, 4H), 2.61 - 2.45 (m, 4H), 2.29 (s, 6H).

### Example 2: Preparation of Compound D

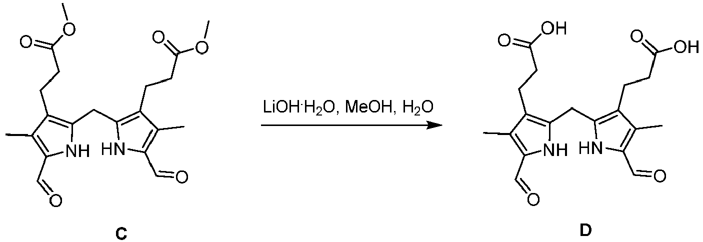

Lithium hydroxide (LiOH·H₂O) (2.75 g, 65.6 mmol, 6.6 equiv.) was added to a mixture of methanol (100 mL) and water (100 mL) as well as Compound C (4.00 g, 9.94 mmol, 1.0 equiv.) of Example 1 above. The mixture was stirred at 25 °C for 16 hours and then diluted with water (100 mL). 1 M hydrochloric acid was added dropwise to the mixture to adjust the pH to 2-3. Thereafter, the precipitate was filtered and dried to yield Compound D corresponding to the compound represented by Formula 1 herein as a purple solid (3.49 g, 9.32 mmol, yield: 94%).

¹H NMR (400 MHz, CDCl₃) δ 12.03 (brs, 2H), 11.51 (s, 2H), 9.48 (s, 2H), 3.91 (s, 2H), 2.54 (overlapped with DMSO-*d*₆'s signal, 4H), 2.18 (s, 6H), 2.06 (t, *J* = 8.0 Hz, 4H).
C₁₉H₂₂N₂OS m/z [M+H]⁺ = 375

### Example 3: Preparation of Compound C1

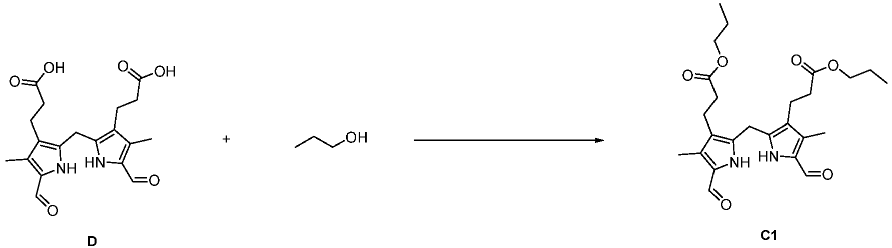

DIPEA (103.56 mg, 0.80 mmol, 3.0 equiv.), HOBt (108.28 mg, 0.80 mmol, 3.0 equiv.) and EDCI (153.61 mg, 0.80 mmol, 3.0 equiv.) were added to a mixture of Compound D (100 mg, 0.26 mmol, 1.0 equiv.) of Example 2 in DMSO (3 mL), and stirred at 25 °C for 30 minutes. 1-propanol (80.26 mg, 1.34 mmol, 5.0 equiv.) was added dropwise to the mixture and stirred for 12 hours. After adding EtOAc (140 mL) to the mixture, it was washed with water (120 mL x 5). The combined organic layer was washed with brine (120 mL), dried over anhydrous Na₂SO₄, filtered and concentrated under reduced pressure. The obtained residue was triturated with MeOH (1 mL) and filtered under reduced pressure to yield Compound C1 corresponding to the compound represented by Formula 1 herein as a pink solid (27 mg, 0.046 mmol, yield: 21%).

¹H NMR (400 MHz, DMSO-*d₆*) δ 11.53 (brs, 2H), 9.47 (s, 2H), 3.91 (t, *J=* 6.8 Hz, 6H), 2.54 (overlapped with DMSO-*d*₆'s signal, 4H), 2.17 (s, 6H), 2.06 (t, *J* = 8.0 Hz, 4H), 1.58 - 1.49 (m, 4H), 0.84 (t, *J* = 7.6 Hz, 3H).

### Example 4: Preparation of Compound C2

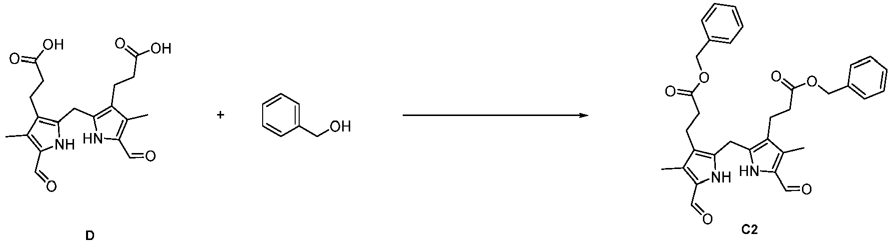

DIPEA (165.56 mg, 1.28 mmol, 3.0 equiv.), HOBt (173.24 mg, 1.28 mmol, 3.0 equiv.) and EDCI (254.78 mg, 1.28 mmol, 3.0 equiv.) were added to a mixture of Compound D (160 mg, 0.42 mmol, 1.0 equiv.) of Example 2 in DMSO (5 mL), and stirred at 25 °C for 30 minutes. After adding benzylalcohol (231.07 mg, 2.14 mmol, 5.0 equiv.) dropwise to the mixture, the mixture was stirred for 12 hours. After adding EtOAc (180 mL) to the mixture, it was washed with water (130 mL x 5). The combined organic layer was washed with brine (130 mL), dried over anhydrous Na₂SO₄, filtered and concentrated under reduced pressure. The obtained residue was triturated with MeOH/MTBE (v/v=1/1, 10 mL) and then filtered under reduced pressure to yield Compound C2 corresponding to the compound represented by Formula 1 herein as a brown solid (110 mg, 0.198 mmol, yield: 47%).

¹H NMR (400 MHz, DMSO-*d₆*) δ 11.53 (s, 2H), 9.45 (s, 2H), 7.37 - 7.26 (m, 10H), 5.03 (s, 4H), 3.87 (s, 2H), 2.53 (overlap with DMSO-*d₆*'s signal, 4H), 2.17 - 2.14 (m, 10H).

### 2. Preparation of compound represented by Formula 2

Compounds (Examples 5 to 28) corresponding to the compound represented by Formula 2 herein were prepared as follows.

### 2.1. Preparation of Compounds Ea-2, Ea-3, Ea-4 and Ea

### Example 5: Preparation of Compound Ea-2

### (5-1) Preparation of Compound Ea-1

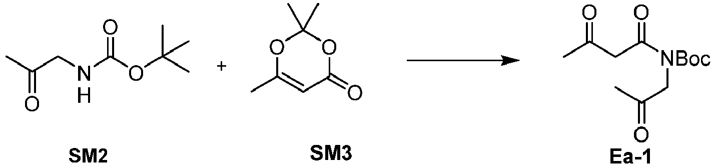

To a mixture of compound SM2 (40.0 g, 231 mmol, 1.0 equiv.) in xylene (241 mL), compound SM3 (32.8 g, 231 mmol, 1.0 equiv.) was added. The mixture was stirred at 150 °C for 10 minutes under nitrogen condition. The mixture was concentrated under reduced pressure condition. The residue was purified by silica gel chromatography to yield Compound Ea-1 in the form of brown oil (51.0 g, 198 mmol, yield: 85%).

¹H NMR (400 MHz, CDCl₃) δ 4.48 (s, 2H), 4.00 (s, 2H), 2.21 (s, 3H), 2.13 (s, 3H), 1.39 (s, 9H).

### (5-2) Preparation of Compound Ea-2

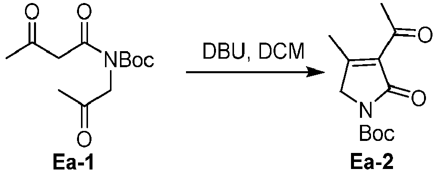

A mixture of DBU (34.8 mL, 233 mmol, 0.5 equiv.) in DCM (34.8 mL) was added dropwise to a mixture of Compound Ea-1 (120 g, 466 mmol, 1.0 equiv.) prepared above in DCM (600 mL) at 0 °C, and stirred for 40 minutes. The reaction was terminated with KH₂PO₄ aqueous solution (480 mL), and the organic layer was extracted with DCM (600 mL x 2) and washed with water (480 mL) and brine (1.2 L). The combined organic layer was dried over anhydrous Na₂SO₄, filtered and concentrated under reduced pressure condition. The residue was dissolved in MTBE (60 mL) and petroleum ether (720 mL) was added thereto at 0 °C. The resulting precipitate was filtered under reduced pressure condition to yield Compound Ea-2 corresponding to the compound represented by Formula 2 herein as a brown solid (90 g, 372 mmol, yield: 80%).

¹H NMR (400 MHz, CDCl₃) δ 4.27 (s, 2H), 2.56 (s, 3H), 2.39 (s, 3H), 1.57 (s, 9H).

### Example 6: Preparation of Compound Ea-3

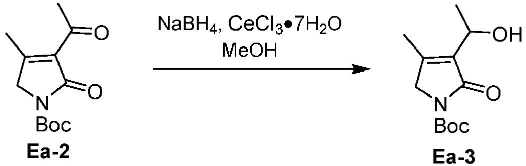

CeCl₃·7H₂O (126g, 338 mmol, 2.0 equiv.) was dissolved in methanol (600 mL) and stirred for 5 minutes. The Compound Ea-2 (53.9 g, 16 9 mmol, 1.0 equiv.) prepared above was added to the above mixture and stirred for 5 minutes. The mixture was then cooled to 0 °C, and NaBH₄ (12.8 g, 338 mmol, 2.0 equiv.) was added slowly over about 1 hour. The mixture was stirred at 0 °C for 3 hours under nitrogen (N₂) condition. 1 M HCl aqueous solution (300 mL) was added to the mixture, followed by extraction with EtOAc (500 mL x 5) and DCM (500 mL x 2) sequentially. The combined organic layer was washed with brine (300 mL), dried over anhydrous Na₂SO₄, filtered and concentrated under reduced pressure condition. The residue was purified by flash silica gel chromatography to yield Compound Ea-3 corresponding to the compound represented by Formula 2 herein in the form of brown oil (23.0 g, 95.3 mmol, yield: 56%).

¹H NMR (400 MHz, CDCl₃) δ 4.72 - 4.64 (m, 1H), 4.13 (s, 2H), 3.49 (d, *J* = 9.6 Hz, 1H), 2.05 (s, 3H), 1.56 (s, 9H), 1.48 (d, *J=* 6.8 Hz, 3H).

### Example 7: Preparation of Compound Ea-4

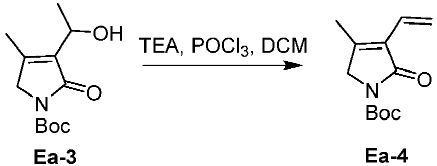

TEA (116 g, 1.14 mol, 12.0 equiv.) was added to a mixture of Compound Ea-3 (23.0 g, 95.3 mmol, 1.0 equiv.) prepared above in DCM (700 mL), and then a mixture of POCl₃ (58.4 g, 381 mmol, 4.0 equiv.) in DCM (180 mL) was added dropwise to the above mixture at 0 °C for 1 hour. The mixture was stirred at 20 °C for 3 hours under nitrogen (N₂) condition and monitored by TLC. The mixture was concentrated under reduced pressure condition and diluted with water (300 mL), followed by extraction with DCM (500 mL x 2). The combined organic layer was washed with brine (200 mL), dried over anhydrous Na₂SO₄, filtered and concentrated under reduced pressure condition to obtain a residue. The residue was purified by flash silica gel chromatography to yield Compound Ea-4 corresponding to the compound represented by Formula 2 herein in the form of yellow solid (8.0 g, 24.2 mmol, yield: 38%).

¹H NMR (400 MHz, CDCl₃) δ 6.45 - 6.38 (m, 1H), 6.31 - 6.26 (m, 1H), 5.46 - 5.42 (m, 1H), 4.15 (s, 2H), 2.11 (s, 3H), 1.56 (s, 9H).

### Example 8: Preparation of Compound Ea

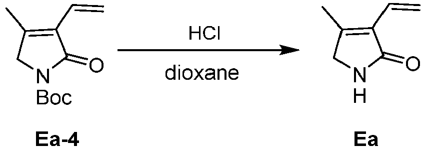

HCl/1,4-dioxane (4 M, 18 mL) was added to a mixture of the Compound Ea-4 (4.0 g, 17.9 mmol) prepared above in EtOAc (18 mL) at 0 °C, then the mixture was stirred at 20 °C for 1 hour. The reaction was terminated by adding NaHCO₃ aqueous solution (80 mL) to the mixture. The mixture was extracted with EtOAc (80 mL x 2) and then with DCM (150 mL). The combined organic layer was washed with brine (100 mL), dried over anhydrous Na₂SO₄, filtered and concentrated thus to yield Compound Ea corresponding to the compound represented by Formula 2 herein in the form of yellow solid (4.6 g, yield: >99 %).

¹H NMR (400 MHz, CDCl₃) δ 6.87 (brs, 1H), 6.48 (dd, *J* = 17.6, 11.6 Hz, 1H), 6.24 (dd, *J=* 18.0, 2.0 Hz, 1H), 5.41 (dd, *J=* 11.6, 2.0 Hz, 1H), 3.86 (s, 2H), 2.09 (s, 3H).

### 2.2. Preparation of Compounds Ea-3a and Ea-2a

### Example 9: Preparation of Compound Ea-3a

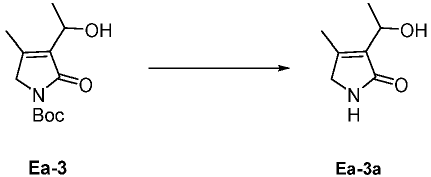

To a mixture of Compound Ea-3 (1.6 g, 6.63 mmol, 1.0 equiv.) prepared above in DCM (10 mL), 4 M HCl/1,4-dioxane (6.63 mL, 26.52 mmol, 4.0 equiv.) was added at 0 °C, and then stirred at 0 °C for 2 hours. After adjusting the pH of the mixture to 7 with NaHCO₃ aqueous solution (100 mL), the organic layer was extracted with DCM (125 mL x 2) and washed with brine (20 mL). The combined organic layer was dried over anhydrous Na₂SO₄, filtered and concentrated under reduced pressure condition to yield Compound Ea-3a corresponding to the compound represented by Formula 2 herein as a brown solid (650 mg, 4.61 mmol, yield: 70%).

¹H NMR (400 MHz, CDCl₃) δ 6.85 (brs, 1H), 5.02 (q, *J* = 6.8 Hz, 1H), 3.85 (s, 2H), 2.17 (s, 3H), 1.82 (d, *J* = 6.8 Hz, 3H)

### Example 10: Preparation of Compound Ea-2a

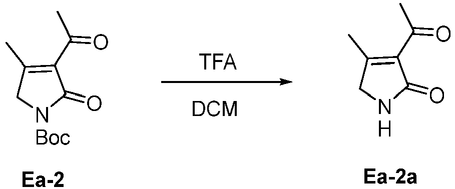

After adding TFA (0.32 mL, 4.18 mmol, 5.0 equiv.) to a mixture of Compound Ea-2 (200 mg, 0.83 mmol, 1.0 equiv.) prepared above in DCM (16 mL), the mixture was stirred at 25 °C for 1 hour. The mixture was adjusted to pH 7 by adding NaHCO₃ aqueous solution (50 mL). After extraction with DCM (50 mL x 3), the combined organic layer was dried over anhydrous Na₂SO₄, filtered and concentrated under reduced pressure condition. After adding DCM (3 mL) to the concentrated filtrate and stirring at 25 °C for 5 minutes, hexane (20 mL) was slowly added dropwise thereto. The precipitate was filtered under reduced pressure to yield Compound Ea-2a corresponding to the compound represented by Formula 2 herein as a yellow solid (70 mg, 0.50 mmol, yield: 60%).

¹H NMR (400 MHz, CDCl₃) δ 7.15 (brs, 1H), 3.97 (s, 2H), 2.55 (s, 3H), 2.36 (s, 3H).

### 2.3. Preparation of Compounds Ea-2b, Ea-3b and Ea-4b

### Example 11: Preparation of Compound Ea-2b

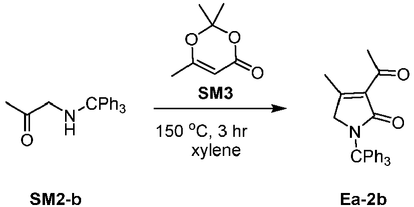

A mixture of compounds SM2-b (1 g, 3.17 mmol, 1.0 equiv.) and SM3 (450.70 mg, 3.17 mmol, 1.0 equiv.) in xylene (15 mL) was stirred at 150 °C for 3 hours. Xylene was removed under reduced pressure, and the residue was purified by silica gel chromatography to yield Compound Ea-2b corresponding to the compound represented by Formula 2 herein as a yellow solid (30 mg, 75.10 µmol, yield: 3%).

¹H NMR (400 MHz, CDCl₃) δ 7.31 - 7.22 (m, 15H), 3.91 (s, 2H), 2.47 (s, 3H), 2.29 (s, 3H).

### Example 12: Preparation of Compound Ea-3b

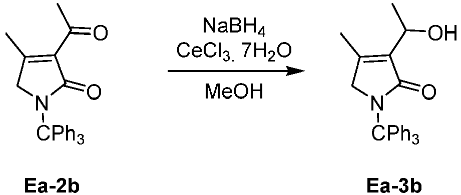

To a mixture of Compound Ea-2b (500 mg, 1.31 mmol, 1.0 equiv.) prepared above and CeCl₃· 7H₂O (976.71 mg, 2.62 mmol, 2.0 equiv.) in methanol (5 mL), NaBH₄ (99.18 mg, 2.62 mmol, 2.0 equiv.) was added at 0 °C. The mixture was stirred at 0 °C for 4 hours. The reaction was terminated with 1 M HCl aqueous solution (10 mL), water (20 mL) was added to the mixture, followed by extraction with EtOAc (30 mL x 2). The combined organic layer was washed with brine (30 mL), dried over anhydrous Na₂SO₄, filtered and concentrated under reduced pressure. The residue was purified by silica gel chromatography to yield Compound Ea-3b corresponding to the compound represented by Formula 2 herein as a yellow solid (102 mg, 0.27 mmol, yield: 20%).

¹H NMR (400 MHz, CDCl₃) δ 7.32 - 7.21 (m, 15H), 4.66 - 4.62 (m, 1H), 3.75 (s, 2H), 1.93 (s, 3H), 1.40 (d, *J* = 6.8 Hz, 3H)

### Example 13: Preparation of Compound Ea-4b

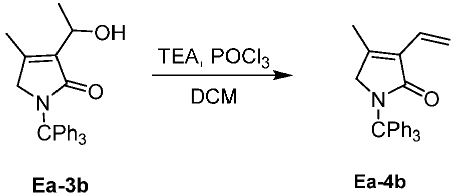

To a mixture of Compound Ea-3b (214 mg, 0.55 mmol, 1.0 equiv.) prepared above and TEA (0.93 mL, 6.70 mmol, 12.0 equiv.) in DCM (5 mL), a mixture of POCl₃ (0.27 mL, 2.23 mmol, 4.0 equiv.) in DCM (2 mL) was added at 0 °C and stirred at 25 °C for 2 hours. The mixture was concentrated under reduced pressure and extracted with DCM (30 mL x 2) after adding water (10 mL). The combined organic layer was washed with brine (20 mL), dried over anhydrous Na₂SO₄, filtered and concentrated under reduced pressure condition. The residue was purified by silica gel chromatography to yield Compound Ea-4b corresponding to the compound represented by Formula 2 herein as a white solid (30 mg, 82.1 µmol, yield: 15%).

¹H NMR(400 MHz, CDCl₃) δ 7.32 - 7.21 (m, 15H), 6.42 (dd, *J* = 18.0, 11.6 Hz, 1H), 6.16 (dd, *J=* 18.0, 2.0 Hz, 1H), 5.34 (dd, *J=* 11.6, 2.0 Hz, 1H), 3.79 (s, 2H), 2.03 (s, 3H)

### 2.4. Preparation of Compounds Ea-2c, Ea-3c and Ea-4c

### Example 14: Preparation of Compound Ea-2c

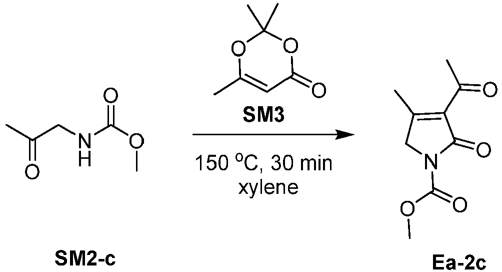

A mixture of compounds SM2-c (598 mg, 4.56 mmol, 1.0 equiv.) and SM3 (648.27 mg, 4.56 mmol, 1.0 equiv.) in xylene (6 mL) was stirred at 150 °C for 30 minutes. Xylene was removed under reduced pressure condition and the residue was purified using silica gel chromatography to yield Compound Ea-2c corresponding to the compound represented by Formula 2 herein as a white solid (484 mg, 2.45 mmol, yield: 53%).

¹H NMR (400 MHz, CDCl₃) δ 4.33 (s, 2H), 3.92 (s, 3H), 2.55 (s, 3H), 2.43 (s, 3H). 2.03 (s, 3H)

### Example 15: Preparation of Compound Ea-3c

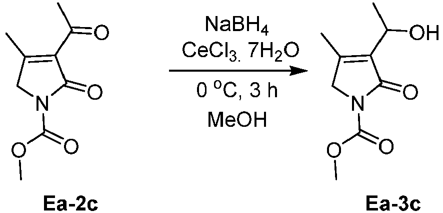

To a mixture of Compound Ea-2c (1.14 g, 5.78 mmol, 1.0 equiv.) prepared above and CeCl₃·7H₂O (4.31 g, 11.56 mmol, 2.0 equiv.) in methanol (10 mL), NaBH₄ (437.44 mg, 11.56 mmol, 2.0 equiv.) was added at 0 °C and stirred at 0 °C for 3 hours. The reaction was terminated with 1 M HCl aqueous solution (20 mL). The mixture was diluted with water (100 mL) and then extracted with EtOAc (150 mL x 2). The combined organic layer was washed with brine (100 mL), dried over anhydrous Na₂SO₄, filtered and concentrated under reduced pressure. The residue was purified by silica gel chromatography to yield Compound Ea-3c corresponding to the compound represented by Formula 2 herein in the form of yellow oil (121 mg, 0.61 mmol, yield: 10%).

¹H NMR (400 MHz, CDCl₃) δ 4.73 - 4.66 (m, 1H), 4.19 (s, 2H), 3.90 (s, 3H), 3.36 (d, *J* = 9.2 Hz, 1H), 2.07 (s, 3H), 1.46 (t, *J=* 6.8 Hz, 3H).

### Example 16: Preparation of Compound Ea-4c

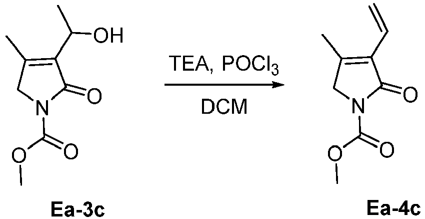

To a mixture of Compound Ea-3c (198 mg, 0.99 mmol, 1.0 equiv.) prepared above and TEA (1.66 mL, 11.93 mmol, 12.0 equiv.) in DCM (5 mL), a mixture of POCl₃ (0.37 mL, 3.98 mmol, 4.0 equiv.) in DCM (3 mL) was added at 0 °C. The mixture was stirred at 25 °C for 2 hours. The mixture was concentrated under reduced pressure condition, diluted with water (20 mL) and extracted with DCM (30 mL x 2). The combined organic layer was washed with brine (20 mL), dried over anhydrous Na₂SO₄, filtered and concentrated under reduced pressure condition. The residue was purified by silica gel chromatography to yield Compound Ea-4c corresponding to the compound represented by Formula 2 herein as a white solid (94 mg, 0.51 mmol, yield: 52%).

¹H NMR (400 MHz, CDCl₃) δ 6.43 (dd, *J=* 17.6, 11.6 Hz, 1H), 6.30 (dd, *J* = 17.6, 2.0 Hz, 1H), 5.47 (dd, *J* = 11.6, 2.0 Hz, 1H), 4.22 (s, 2H), 3.91 (s, 3H), 2.13 (s, 3H).

### 2.5. Preparation of Compounds Ea-2d, Ea-3d and Ea-4d

### Example 17: Preparation of Compound Ea-2d

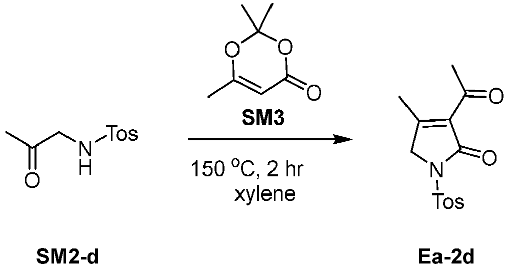

A mixture of compounds SM2-d (5.0 g, 22.0 mmol, 1.0 equiv.) and SM3 (3.13 g, 22.0 mmol, 1.0 equiv.) in xylene (40 mL) was stirred at 150 °C for 2 hours under nitrogen condition. The mixture was concentrated under reduced pressure condition, and the residue was purified by silica gel chromatography to yield Compound Ea-2d corresponding to the compound represented by Formula 2 herein as a white solid (1.41 g, 4.81 mmol, yield: 22%).

¹H NMR (400 MHz, CDCl₃) δ 7.98 (d, *J* = 8.4 Hz, 2H), 7.38 (d, *J* = 8.0 Hz, 2H), 4.43 (s, 2H), 2.50 (s, 3H), 2.47 (s, 3H), 2.40 (s, 3H).

### Example 18: Preparation of Compound Ea-3d

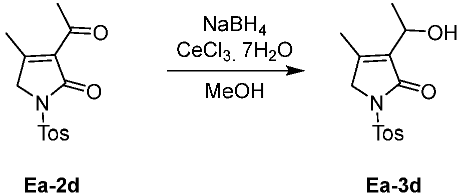

To a mixture of CeCl₃·7H₂O (2.82 g, 7.57 mmol, 2.0 equiv.) in methanol (20 mL), Compound Ea-2d (1.11 g, 3.78 mmol, 1.0 equiv.) prepared above and NaBH₄ (99.18 mg, 2.62 mmol, 2.0 equiv.) were added. The mixture was stirred at 0 °C for 3 hours. The reaction was terminated by adding 1 M HCl aqueous solution (10 mL) to the reactant, followed by extraction with EtOAc (50 mL x 2). The combined organic layer was washed with brine (20 mL), dried over anhydrous Na₂SO₄, filtered and concentrated under reduced pressure. The residue was purified by silica gel chromatography to yield Compound Ea-3d corresponding to the compound represented by Formula 2 herein as a colorless solid (681 mg, 2.31 mmol, yield: 61%).

¹H NMR (400 MHz, CDCl₃) δ 7.94 (d, *J* = 8.4 Hz, 2H), 7.35 (d, *J* = 8.0 Hz, 2H), 4.65 - 4.58 (m, 1H), 4.26 (q, *J=* 16.0 Hz, 2H), 3.02 (d, *J=* 8.8 Hz, 2H), 2.44 (s, 3H), 2.06 (s, 3H), 1.42 (d, *J* = 6.8 Hz, 3H).
C₁₄H₁₇NO₄S m/z [M+H]⁺ = 295.9

### Example 19: Preparation of Compound Ea-4d

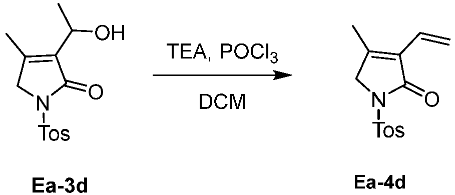

To a mixture of Compound Ea-3d (680 mg, 2.30 mmol, 1.0 equiv.) prepared above in DCM (20 mL), TEA (3.85 mL, 27.63 mmol, 12.0 equiv.) and POCl₃ (0.86 mL, 9.21 mmol, 4.0 equiv.) were added, and stirred at 0°C for 2 hours under nitrogen condition. The mixture was concentrated under reduced pressure and extracted with DCM (50 mL x 4) after adding water (50 mL). The combined organic layer was washed with brine (20 mL), dried over anhydrous Na₂SO₄, filtered and concentrated under reduced pressure condition. The residue was purified by silica gel chromatography to yield Compound Ea-4d corresponding to the compound represented by Formula 2 herein as a white solid (276 mg, 0.99 mmol, yield: 43%).

¹H NMR (400 MHz, CDCl₃) δ 7.97 (d, *J* = 8.4 Hz, 2H), 7.34 (d, *J* = 8.4 Hz, 2H), 6.33 (dd, *J* = 18.0, 11.6 Hz, 1H), 6.22 (dd, *J* = 18.0, 2.0 Hz, 1H), 5.43 (dd, *J* = 11.2, 2.0 Hz, 1H), 4.30 (s, 2H), 2.43 (s, 3H), 2.10 (s, 3H).

### 2.6. Preparation of Compounds Ea-2e, Ea-3e and Ea-4e

### Example 20: Preparation of Compound Ea-2e

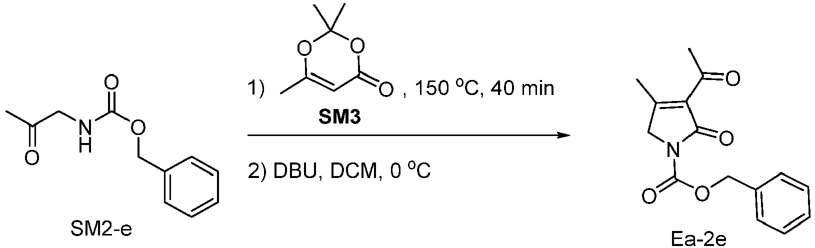

A mixture of compounds SM2-e (360 mg, 1.74 mmol, 1.0 equiv.) and SM3 (321 mg, 2.26 mmol, 1.0 equiv.) was stirred at 150 °C for 40 minutes. After completion of the reaction, the residue was purified using silica gel chromatography. To a mixture of the purified residue in DCM (5 mL), DBU (130 mg) was added at 0 °C. After stirring the mixture at 0 °C for 40 minutes, the reaction was terminated with K₂HPO₄ aqueous solution (100 mL). The mixture was extracted with DCM (100 mL x 2), washed with brine and dried over anhydrous Na₂SO₄. The dried organic layer was filtered and concentrated. The residue was purified by silica gel chromatography to yield Compound Ea-2e corresponding to the compound represented by Formula 2 herein as a yellow solid (280 mg, 1.02 mmol, yield: 60%).
C₁₅H₁₅NO₄ m/z [M+H]⁺ = 274

### Example 21: Preparation of Compound Ea-3e

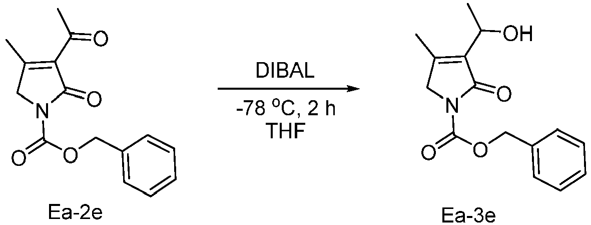

To a mixture of Compound Ea-2e (100 mg, 0.380 mmol, 1.0 equiv.) prepared above in THF (2 mL), DIBAL (660 mL, 0.680 mmol, 2.0 equiv.) was added at -78°C and stirred at -78°C for 2 hours. The reaction was terminated by adding NH₄Cl aqueous solution (50 mL) to the mixture, followed by extraction with DCM (100 mL x 2). Then, the combined organic layer was washed with brine (100 mL) and then dried over anhydrous Na₂SO₄, filtered and concentrated under reduced pressure condition. The residue was purified by silica gel chromatography to yield Compound Ea-3e corresponding to the compound represented by Formula 2 herein in the form of yellow oil (22 mg, 0.08 mmol, yield: 21%).
C₁₅H₁₇NO₄ m/z [M+H]⁺ = 276

### Example 22: Preparation of Compound Ea-4e

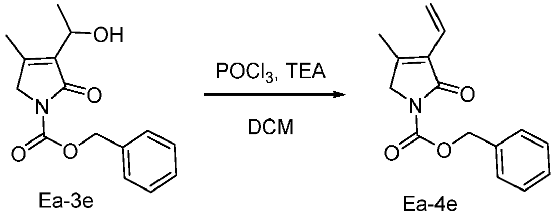

To a mixture of Compound Ea-3e (22.0 mg, 0.08 mmol, 1.0 equiv.) prepared above in DCM (1.0 mL), TEA (111 µL, 0.8 mmol, 10.0 equiv.) and POCl₃ (11 µL, 0.24 mmol, 3.0 equiv.) were added sequentially at 0 °C. The mixture was stirred at 20 °C for 3 hours under nitrogen (N₂) condition. The mixture was concentrated under reduced pressure condition, diluted with water (50 mL) and extracted with DCM (50 mL x 2). The combined organic layer was washed with brine (100 mL), dried over anhydrous Na₂SO₄, filtered and concentrated under reduced pressure condition to obtain a residue. The residue was purified by flash silica gel chromatography to yield Compound Ea-4e corresponding to the compound represented by Formula 2 herein in the form of white solid (7.0 mg, 0.027 mmol, yield: 34%).

¹H NMR (400 MHz, CDCl₃) δ 7.45 - 7.31 (m, 5H), 6.41 (dd, *J* = 17.7, 11.5 Hz, 1H), 6.30 (dd, *J=* 17.8, 2.1 Hz, 1H), 5.49 - 5.43 (m, 1H), 4.21 (s, 2H), 2.10 (s, 3H).

### 2.7. Preparation of Compounds Eb-5 and Eb

### Example 23: Preparation of Compound Eb-5

### (23-1) Preparation of Compound Eb-2

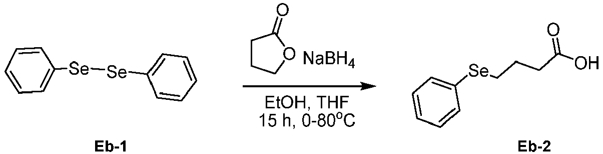

To a mixture of Compound Eb-1 (30.0 g, 96.1 mmol, 1.0 equiv.) dissolved in EtOH (300 mL), NaBH₄ (7.27 g, 192 mmol, 2.0 equiv.) was added slowly at 0°C, and stirred at 0 °C for 1 hour under a nitrogen environment. Further, tetrahydrofuran-2-one (16.6 g, 192 mmol, 2.0 equiv.) dissolved in THF (20 mL) was added to the mixture. The mixture was stirred at 80 °C for 15 hours under a nitrogen environment. After cooling the mixture to 25 °C, it was diluted with water (200 mL), then adjusted to pH 2 with 2 M HCl aqueous solution, followed by extraction with DCM (75 mL x 3). The combined organic layer was washed with brine (50 mL), dried over anhydrous Na₂SO₄, filtered and concentrated under reduced pressure condition. The residue was purified by flash silica gel chromatography to yield Compound Eb-2 as a white solid (15.0 g, 61.7 mmol, yield: 64%).

¹H NMR (400 MHz, CDCl₃) δ 7.53 - 7.51 (m, 2H), 7.29 - 7.26 (m, overlap with CDCl₃'s signal, 3H), 2.97 (t, *J* = 7.2 Hz, 2H), 2.53 (t, *J* = 7.2 Hz, 2H), 2.06 - 1.99 (m, 2H).

### (23-2) Preparation of Compound Eb-3

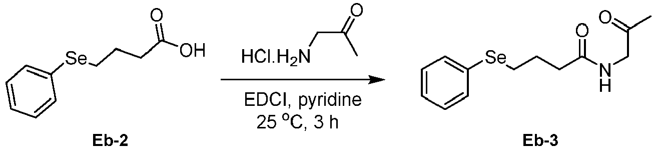

To a mixture of Compound Eb-2 (14.0 g, 57.6 mmol, 1.0 equiv.) prepared above in pyridine (100 mL), EDCI (13.2 g, 69.1 mmol, 1.2 equiv.) and 1-aminopropan-2-one hydrochloride (6.31 g, 57.6 mmol, 1.0 equiv.) were added, and the mixture was stirred at 25 °C for 3 hours under a nitrogen environment. Water (50 mL) was added to the mixture, followed by extraction with DCM (60 mL x 3). The combined organic layer was washed with brine (50 mL), dried over anhydrous Na₂SO₄, filtered and concentrated under reduced pressure condition. The residue was purified by flash silica gel chromatography to yield Compound Eb-3 as a light yellow solid (6.30 g, 21.1 mmol, yield: 37%).

¹H NMR (400 MHz, CDCl₃) δ 7.54 - 7.45 (m, 2H), 7.35 - 7.20 (m, overlap with CDCl₃'s signal, 3H), 6.23 (brs, 1H), 4.20 - 4.10 (m, 2H), 2.96 (t, *J* = 7.2 Hz, 2H), 2.39 (t, *J* = 7.2 Hz, 2H), 2.20 (s, 3H), 2.07 - 2.00 (m, 2H).

### (23-3) Preparation of Compound Eb-4

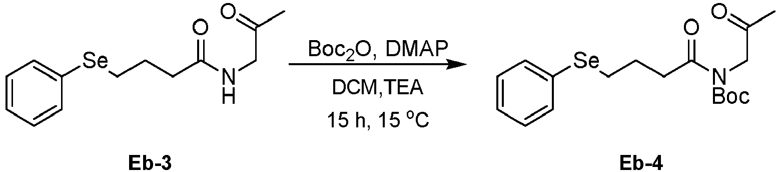

To a mixture of Compound Eb-3 (4.49 g, 15.1 mmol, 1.0 equiv.) prepared above in DCM (30 mL), Boc₂O (6.57 g, 30.1 mmol, 2.0 equiv.), TEA (1.52 g, 15.1 mmol, 1.0 equiv.) and DMAP (1.84 g, 15.1 mmol, 1.0 equiv.) were added, and the mixture was stirred at 15 °C for 15 hours under nitrogen condition. After removing the solvent under reduced pressure condition, the residue was purified by flash silica gel chromatography to yield Compound Eb-4 as a bright yellow solid (5.40 g, 13.6 mmol, yield: 90%).

¹H NMR (400 MHz, CDCl₃) δ 7.53 - 7.49 (m, 2H), 7.29 - 7.22 (m, overlap with CDCl₃'s signal, 3H), 4.51 (2H, s), 3.10 (t, *J* = 7.2 Hz, 2H), 2.97 (t, *J* = 7.2 Hz, 2H), 2.16 (s, 3H), 2.09 - 2.01 (m, 2H), 1.49 (s, 9H).

### (23-4) Preparation of Compound Eb-5

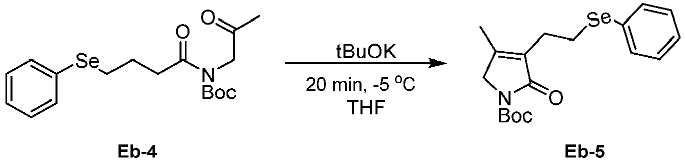

To a mixture of Compound Eb-4 (1.48 g, 3.72 mmol, 1.0 equiv.) prepared above in THF (40 mL), t-BuOK (7.43 mL, 2.0 equiv., 1 M in THF) was added at -5 °C under nitrogen condition, and stirred at -5 °C for 20 minutes. After adding cold water (70 mL) to this mixture, it was extracted with DCM (50 mL x 4). The combined organic layer was washed with brine (20 mL), dried over anhydrous Na₂SO₄, filtered and concentrated under reduced pressure condition. The residue was purified by flash silica gel chromatography to yield Compound Ea-5 corresponding to the compound represented by Formula 2 herein in the form of colorless and transparent oil (413 mg, 1.09 mmol, yield: 29%).

¹H NMR (400 MHz, CDCl₃) δ 7.51 - 7.46 (m, 2H), 7.29 - 7.20 (m, overlap with CDCl₃'s signal, 3H), 3.98 (s, 2H), 3.15 (t, *J* = 7.2 Hz, 2H), 2.69 (t, *J* = 7.2 Hz, 2H), 1.94 (s, 3H), 1.56 (s, 9H).

### Example 24: Preparation of Compound Eb

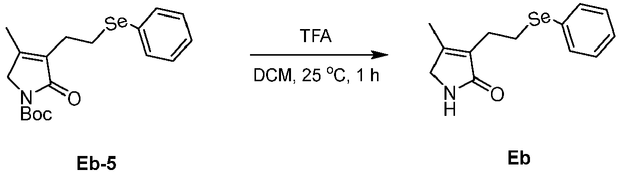

To a mixture of Compound Eb-5 (697 mg, 1.83 mmol, 1.0 equiv.) prepared above in DCM (10 mL), TFA (627 mg, 5.50 mmol, 3.0 equiv.) was added and stirred at 25 °C for 1 hour under nitrogen condition. The mixture was adjusted to pH 8 with NaHCO₃ aqueous solution (20 mL) and then extracted with DCM (20 mL x 3). The combined organic layer was washed with brine (20 mL), dried over anhydrous Na₂SO₄, filtered and concentrated under reduced pressure condition to yield Compound Eb corresponding to the compound represented by Formula 2 herein in the form of orange oil (595 mg, yield: >99%).

¹H NMR (400 MHz, CDCl₃) δ 7.50 - 7.35 (m, 2H), 7.20 - 7.11 (m, overlap with CDCl₃'s signal, 3H), 6.93 (brs, 1H), 3.66 (s, 2H), 3.07 (t, *J* = 7.2 Hz, 2H), 2.62 (t, *J* = 7.2 Hz, 2H), 1.85 (s, 3H).

### 2.8. Preparation of Compounds Ed-11 and Ed

### Example 25: Preparation of Compound Ed-11

### (25-1) Preparation of Compound Ed-2

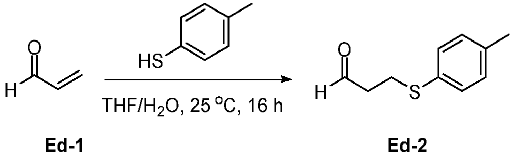

To a mixture of 4-methylbenzenethiol (36.9 g, 297 mmol, 0.83 equiv.) in THF (300 mL) and water (150 mL), Ed-1 (20.1 g, 358 mmol, 1.0 equiv.) was slowly added at 0 °C, and stirred at 25 °C for 16 hours under nitrogen condition. NaHCO₃ aqueous solution (200 mL) was added to the mixture, followed by extraction with EtOAc (200 mL x 2). The combined organic layer was washed with brine (50 mL x 2), dried over anhydrous Na₂SO₄, filtered and concentrated under reduced pressure condition to yield Compound Ed-2 in the form of yellow oil (59.4 g, 330 mmol, yield: 92 %).

¹H NMR (400 MHz, CDCl₃) δ 9.74 (s, 1H), 7.27 (d, *J* = 8.0 Hz, 2H), 7.11 (d, *J* = 7.6 Hz, 2H), 3.13 (t, *J=* 7.2 Hz, 2H), 2.75 - 2.71 (m, 2H), 2.32 (s, 3H).

### (25-2) Preparation of Compound Ed-3

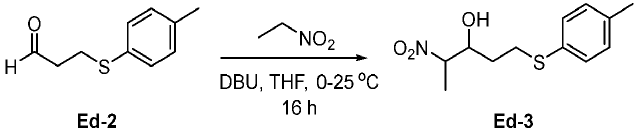

To a mixture of Compound Ed-2 (58 g, 322 mmol, 1.0 equiv.) prepared above and DBU (4.90 g, 32.2 mmol, 0.1 equiv.) in THF (400 mL), a mixture of 1-nitroethane (24.0 g, 322 mmol, 1.0 equiv.) in THF (50 mL) was added at 0 °C, and stirred at 25 °C for 16 hours. The mixture was diluted with water (200 mL) then extracted with EtOAc (400 mL x 2). The combined organic layer was washed with brine (200 mL), dried over anhydrous Na₂SO₄, filtered and concentrated under reduced pressure condition. The residue was purified by flash silica gel chromatography to yield Compound Ed-3 in the form of yellow oil (51.7 g, 202 mmol, yield: 63%).

¹H NMR (400 MHz, CDCl₃) δ 7.28 (d, *J* = 7.6 Hz, 2H), 7.13 (d, *J* = 8.0 Hz, 2H), 4.54 - 4.46 (m, 1H), 4.15 - 4.12 (m, 1H), 3.15 - 3.08 (m, 1H), 3.03 - 2.96 (m, 1H), 2.33 (s, 3H), 1.80 - 1.64 (m, 2H), 1.53 (t, *J* = 8.0 Hz, 3H).

### (25-3) Preparation of Compound Ed-4

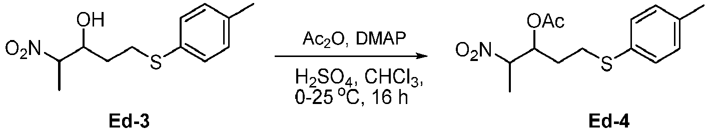

To a mixture of Compound Ed-3 (51.7 g, 202 mmol, 1.0 equiv.) prepared above and H₂SO₄ (199 mg, 2.02 mmol, 0.01 equiv.) in chloroform (500 mL), acetic anhydride (31.0 g, 304 mmol, 1.5 equiv.) was added slowly at 0 °C and stirred at 25 °C for 16 hours. The reaction was terminated by adding NaHCO₃ aqueous solution (100 mL) to the mixture, followed by extraction with DCM (50 mL x 4). The combined organic layer was washed with brine (50 mL x 2), dried over anhydrous Na₂SO₄, filtered and concentrated under reduced pressure condition to yield Compound Ed-4 in the form of brown oil (65.5 g, yield: >99%).

¹H NMR (400 MHz, CDCl₃) δ 7.27 (d, *J* = 8.4 Hz, 2H), 7.12 (d, *J* = 8.0 Hz, 2H), 5.45 - 5.40 (m, 1H), 4.75 - 4.66 (m, 1H), 2.98 - 2.78 (m, 2H), 2.33 (s, 3H), 2.07 (d, *J* = 8.8 Hz, 3H), 1.99 - 1.80 (m, 2H), 1.49 (dd, *J=* 6.8, 2.0 Hz, 3H).

### (25-4) Preparation of Compound Ed-5

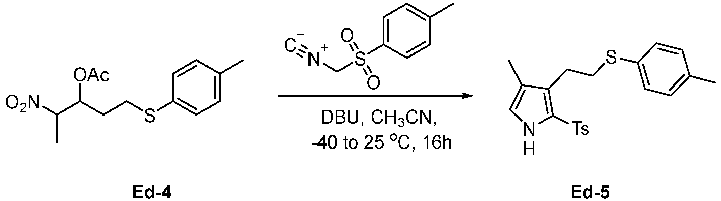

To a mixture of Compound Ed-4 (3.61 g, 18.5 mmol, 1.0 equiv.) prepared above and DBU (5.63 g, 37.0 mmol, 2.0 equiv.) in ACN (50 mL), a mixture of TosMIC (5.00 g, 16.8 mmol, 0.9 equiv.) in ACN (10 mL) was added dropwise at -40 °C under a nitrogen environment, and stirred at 25 °C for 16 hours under a nitrogen environment. The mixture was diluted with water (100 mL) then extracted with EtOAc (100 mL x 2). The combined organic layer was washed with brine (100 mL), dried over anhydrous Na₂SO₄, filtered and concentrated under reduced pressure condition. The residue was purified by flash silica gel chromatography to yield Compound Ed-5 in the form of red oil (3.47 g, 9.00 mmol, yield: 53%).

¹H NMR (400 MHz, CDCl₃) δ 8.99 (s, 1H), 7.65 (d, *J=* 8.0 Hz, 2H), 7.31 (d, *J* = 8.0 Hz, 2H), 7.21 (d, *J* = 8.0 Hz, 2H), 7.14 (d, *J* = 8.0 Hz, 2H), 6.69 (d, *J* = 2.0 Hz, 1H), 2.87 - 2.81 (m, 4H), 2.37 (s, 3H), 2.35 (s, 3H), 1.93 (s, 3H).

### (25-5) Preparation of Compound Ed-6

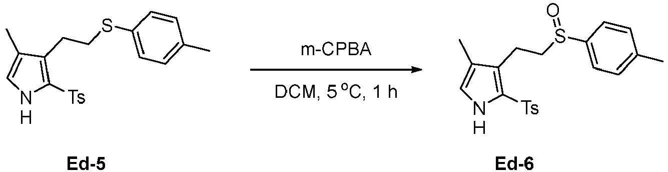

To a mixture of Compound Ed-5 (10.0 g, 25.9 mmol, 1.0 equiv.) prepared above in DCM (40 mL), m-CPBA (5.27 g, 25.9 mmol, 85% purity, 1.0 equiv.) was added dropwise at 5 °C under a nitrogen environment, and stirred at 5 °C for 1 hour. The reaction was terminated by adding Na₂SO₃ aqueous solution (100 mL) to the mixture, and extracted with DCM (50 mL x 2). The combined organic layer was washed with brine (100 mL), dried over anhydrous Na₂SO₄, filtered and concentrated under reduced pressure condition to obtain a residue. The residue was purified by flash silica gel chromatography to yield Compound Ed-6 in the form of white solid (5.0 g, 12.5 mmol, yield: 48%).

¹H NMR (400 MHz, CDCl₃) δ 9.20 (s, 1H), 7.57 - 7.52 (m, 4H), 7.36 (d, *J* = 8.4 Hz, 2H), 7.21 (d, *J=* 8.0 Hz, 2H), 6.69 (d, *J=* 2.8 Hz, 1H), 2.97 - 2.93 (m, 2H), 2.89 - 2.69 (m, 2H), 2.45 (s, 3H), 2.39 (s, 3H), 1.94 (s, 3H).

### (25-6) Preparation of Compound Ed-7

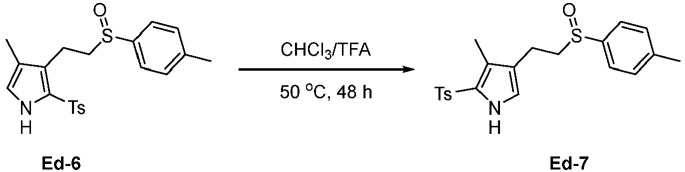

A mixture of Compound Ed-6 (5.0 g, 12.5 mmol, 1.0 equiv.) prepared above and TFA (5 mL) in chloroform (45 mL) was stirred at 50 °C for 48 hours under a nitrogen environment. The reaction was terminated by adding water (100 mL) to the mixture and then extracted with DCM (100 mL x 2). The combined organic layer was washed with brine (50 x 2 mL), dried over anhydrous Na₂SO₄, filtered and concentrated under reduced pressure condition to obtain a residue. The residue was purified by flash silica gel chromatography to yield Compound Ed-7 in the form of white solid (1.90 g, 4.68 mmol, yield: 37%).

¹H NMR (400 MHz, CDCl₃) δ 9.02 (s, 1H), 7.75 (d, *J=* 8.4 Hz, 2H), 7.49 (d, *J* = 8.4 Hz, 2H), 7.33 - 7.27 (m, 4H), 6.74 (d, *J=* 2.8 Hz, 1H), 2.91 - 2.64 (m, 4H), 2.42 (s, 3H), 2.40 (s, 3H), 2.10 (s, 3H).

### (25-7) Preparation of Compound Ed-8

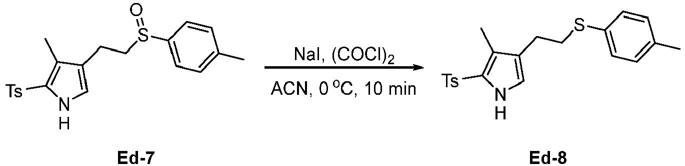

To a mixture of Compound Ed-7 (3 g, 7.47 mmol, 1.0 equiv.) prepared above in ACN (48 mL), NaI (2.82 g, 18.67 mmol, 2.5 equiv.) was added at 0 °C and stirred for 10 minutes. (COCl)₂ (0.77 ml, 9.38 mmol, 1.2 equiv.) was added dropwise to the mixture at 0 °C and stirred for 10 minutes under the same condition. The reaction was terminated by adding water (30 mL) to the mixture, followed by extraction with EtOAc (50 mL x 2). The combined organic layer was washed with brine (100 mL), dried over anhydrous Na₂SO₄, filtered and concentrated under reduced pressure condition. The residue was purified by silica gel chromatography to yield Compound Ed-8 in the form of brown solid (2.21 g, 5.71 mmol, yield: 76%).

¹H NMR (400 MHz, CDCl₃) δ 8.83 (s, 1H), 7.76 (d, *J=* 8.0 Hz, 2H), 7.29 (d, *J* = 8.0 Hz, 2H), 7.23 (d, *J=* 8.4 Hz, 2H), 7.09 (d, *J=* 8.0 Hz, 2H), 6.75 (d, *J=* 2.8 Hz, 1H), 3.00 - 2.96 (m, 2H), 2.65 (t, *J=* 8.0 Hz, 2H), 2.41 (s, 3H), 2.32 (s, 3H), 2.12 (s, 3H).

### (25-8) Preparation of Compound Ed-9

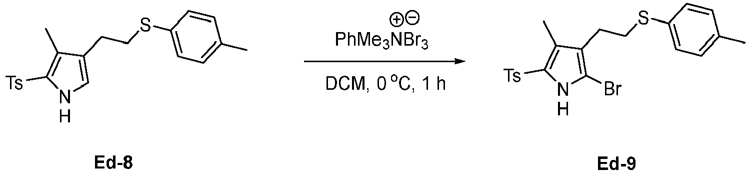

To a mixture of Compound Ed-8 (2.2 g, 5.71 mmol, 1.0 equiv.) prepared above in DCM (62 mL), PhMe₃NBr₃ (2.36 g, 5.71 mmol, 1.0 equiv.) was added at 0 °C and stirred for 1 hour. NaHSO₃ aqueous solution (30 mL) was added to the mixture, followed by extraction with DCM (50 mL x 2). The combined organic layer was washed with brine (100 mL), dried over anhydrous Na₂SO₄, filtered and concentrated under reduced pressure condition. The residue was purified by silica gel chromatography to yield Compound Ed-9 in the form of brown solid (2.65 g, 5.23 mmol, yield: 99%).

¹H NMR (400 MHz, CDCl₃) δ 8.93 (s, 1H), 7.76 (d, *J=* 8.0 Hz, 2H), 7.30 (d, *J* = 8.4 Hz, 2H), 7.25 (d, *J* = 8.0 Hz, 2H), 7.09 (d, *J* = 8.0 Hz, 2H), 2.92 (t, *J* = 7.6 Hz, 2H), 2.62 (t, *J* = 7.6 Hz, 2H), 2.42 (s, 3H), 2.32 (s, 3H), 2.13 (s, 3H).

### (25-9) Preparation of Compound Ed-10

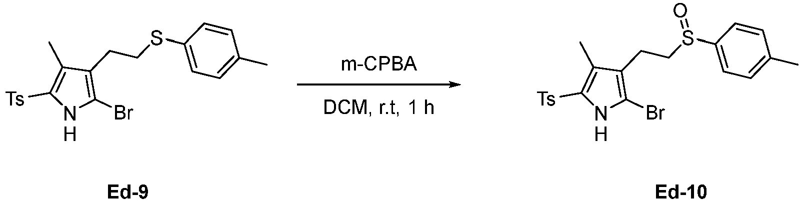

To a mixture of Compound Ed-9 (2.59 g, 5.57 mmol, 1.0 equiv.) prepared above in DCM (50 mL), m-CPBA (1.05 g, 6.13 mmol, 1.1 equiv.) was added at 0 °C and stirred at 25 °C for 1 hour. NaHSO₃ aqueous solution (30 mL) was added to the mixture, followed by extraction with DCM (60 mL x 2). The combined organic layer was washed with brine (100 mL), dried over anhydrous Na₂SO₄, filtered and concentrated under reduced pressure condition. DCM/Hexanes were added to the residue, and the precipitate was filtered to yield Compound Ed-10 as a white solid (2.9 g, yield: >99%).

¹H NMR (500 MHz, CDCl₃) δ 9.00 (s, 1H), 7.68 (d, *J=* 8.0 Hz, 2H), 7.43 (d, *J* = 7.9 Hz, 2H), 7.24 (dd, *J* = 12.2, 8.0 Hz, 4H), 2.86 - 2.78 (m, 1H), 2.78 - 2.67 (m, 2H), 2.58 - 2.48 (m, 1H), 2.34 (s, 3H), 2.33 (s, 3H), 2.04 (s, 3H).

### (25-10) Preparation of Compound Ed-11

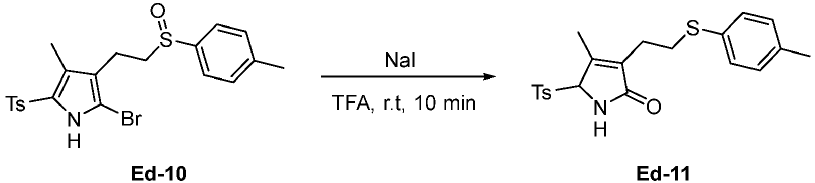

A mixture of Compound Ed-10 (1.0 g, 2.08 mmol, 1.0 equiv.) prepared above and TFA (1.5 mL) was stirred at 25 °C for 30 min under nitrogen condition. NaI (1.56 g, 10.4 mmol, 5.0 equiv.) was added and stirred at 25 °C for 10 min, neutralized by adding K₂CO₃ aqueous solution at 0 °C and extracted with DCM (150 mL x 2). The combined organic layer was dried over anhydrous Na₂SO₄, filtered and concentrated under reduced pressure condition. The residue was purified by silica gel chromatography to yield Compound Ed-11 corresponding to the compound represented by Formula 2 herein as a dark green solid (550 mg, 1.37 mmol, yield: 66%).

¹H NMR (500 MHz, CDCl₃) δ 7.59 (d, *J* = 8.2 Hz, 2H), 7.23 (d, *J* = 8.0 Hz, 2H), 7.10 (d, *J* = 8.1 Hz, 2H), 7.04 (d, *J* = 7.9 Hz, 2H), 6.30 (s, 1H), 2.71 - 2.65 (m, 1H), 2.41 - 2.32 (m, 2H), 2.30 (s, 3H), 2.25 (s, 3H), 2.21 - 2.14 (m, 1H), 2.05 (s, 3H).

### Example 26: Preparation of Compound Ed

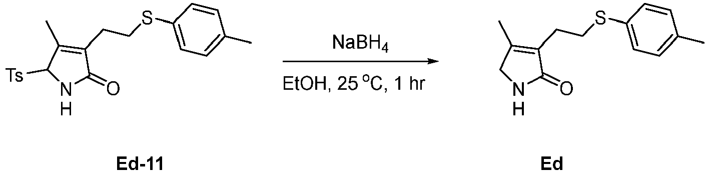

To a mixture of Compound Ed-11 (0.55 g, 1.37 mmol, 1.0 equiv.) prepared above in EtOH (50 mL), NaBH₄ (67 mg, 1.78 mmol, 1.3 equiv.) was added at 0 °C and stirred at 25 °C for 1 hour. NH₄Cl aqueous solution (30 mL) was added to the mixture and extracted with DCM (60 mL x 2). The combined organic layer was washed with brine (100 mL), dried over anhydrous Na₂SO₄, filtered and concentrated under reduced pressure condition. DCM/Hexane were added to the residue, and the precipitated white solid was filtered to yield Compound Ed corresponding to the compound represented by Formula 2 herein (305 mg, 1.23 mmol, yield: 90%).

¹H NMR (500 MHz, DMSO-*d₆*) δ 7.92 (s, 1H), 7.25 (d, *J* = 7.9 Hz, 2H), 7.13 (d, *J* = 7.9 Hz, 2H), 3.70 (s, 2H), 3.01 (t, *J* = 7.2 Hz, 2H), 2.40 (t, *J* = 6.8 Hz, 2H), 2.26 (s, 3H), 1.86 (s, 3H).

### 2.9. Preparation of Compounds Ee-5 and Ee

### Example 27: Preparation of Compound Ee-5

### (27-1) Preparation of Compound Ee-3

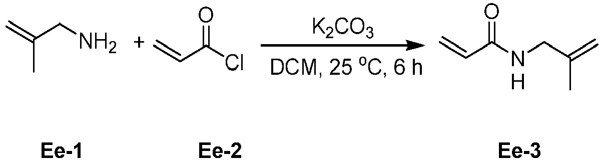

To a mixture of Compound Ee-1 (20.0 g, 281 mmol, 1.0 equiv.) in DCM (300 mL), K₂CO₃ (81.6 g, 591 mmol, 2.1 equiv.) was added at 0 °C and stirred for 15 minutes. Then, Compound Ee-2 (25.5 g, 281 mmol, 1.0 equiv.) was added at 0 °C and stirred at 25 °C for 6 hours. The mixture was filtered under reduced pressure condition, and the residue was purified through silica gel chromatography to yield Compound Ee-3 (35.0 g, 279 mmol, yield: 99%).

¹H NMR (400 MHz, CDCl₃) δ 6.28 (dd, *J* = 16.8, 1.6 Hz, 1H), 6.16 (dd, *J* = 16.8, 10.0 Hz, 1H), 6.15 (brs, 1H), 5.63 (dd, *J* = 10.4, 1.6 Hz, 1H), 4.83 (s, 2H), 3.86 (d, *J* = 6.0 Hz, 2H), 1.72 (s, 3H).

### (27-2) Preparation of Compound Ee-4

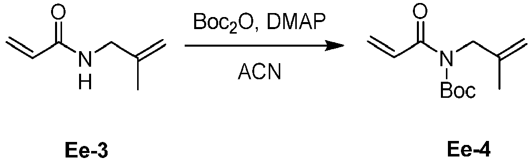

To a mixture of Compound Ee-3 (36.0 g, 288 mmol, 1.0 equiv.) prepared above and Boc₂O (75.3 g, 345 mmol, 1.2 equiv.) in ACN (300 mL), a mixture of DMAP (3.51 g, 28.8 mmol, 0.1 equiv.) in ACN (50 mL) was added at 25 °C and stirred for 16 hours. After concentration under reduced pressure condition to remove ACN, the residue was diluted with water (100 mL), followed by extraction with EtOAc (150 mL x 2). The combined organic layer was washed with brine (100 mL) and dried over anhydrous Na₂SO₄, filtered and concentrated under reduced pressure condition. The residue was purified by silica gel chromatography to yield Compound Ee-4 as a yellow solid (46.8 g, 207 mmol, yield: 72%).

¹H NMR (400 MHz, CDCl₃) δ 7.05 (dd, *J* = 16.8, 10.4 Hz, 1H), 6.34 (dd, *J* = 16.8, 1.6 Hz, 1H), 5.71 (dd, *J* = 10.4, 1.6 Hz, 1H), 4.83 (s, 1H), 4.70 (s, 1H), 4.26 (s, 2H), 1.73 (s, 3H), 1.50 (s, 9H).

### (27-3) Preparation of Compound Ee-5

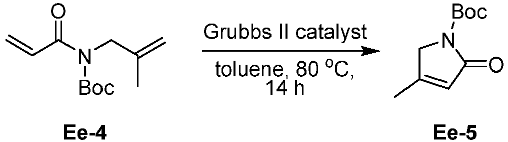

To a mixture of Compound Ee-4 (5.0 g, 22.2 mmol, 1.0 equiv.) prepared above in toluene (50 mL), Grubbs second-generation catalyst (1.32 g, 1.55 mmol, 0.07 equiv.) was added and stirred at 80 °C for 14 hours. The mixture was concentrated under reduced pressure condition to remove toluene. The residue was purified by reverse phase column to yield Compound Ee-5 corresponding to the compound represented by Formula 2 herein as a yellow solid (2.14 g, 10.9 mmol, yield: 49%).

¹H NMR (400 MHz, CDCl₃) δ 5.83 (s, 1H), 4.19 (s, 2H), 2.08 (s, 3H), 1.53 (s, 9H).

### Example 28: Preparation of Compound Ee

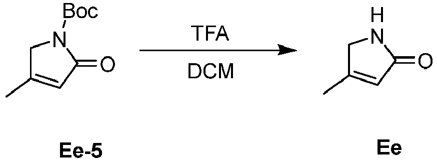

TFA (0.3 mL, 6.08 mmol, 3.0 equiv.) was added to a mixture of Compound Ee-5 (0.4 g, 2.02 mmol, 1.0 equiv.) prepared above in DCM (4 mL) and stirred at 25 °C for 3 hours. The pH of the mixture was adjusted to 7 with NaHCO₃ aqueous solution (10 mL) and extracted with DCM (20 mL x 2). The combined organic layer was dried over anhydrous Na₂SO₄, filtered and concentrated under reduced pressure condition to yield Compound Ee corresponding to the compound represented by Formula 2 herein as a white solid (0.12 g, 1.23 mmol, yield: 61%).

¹H NMR (400 MHz, CDCl₃) δ 6.46 (s, 1H), 5.85 (s, 1H), 3.92 (s, 2H), 2.09 (s, 3H).

### 3. Preparation of the compound represented by Formula 3

Compounds represented by Formula 3 (Examples 29 to 76), respectively, were prepared by coupling the compound represented by Formula 1 and the compound represented by Formula 2 herein.

### 3.1. Preparation of Compound F-3a by coupling of Compound D and Compound Ea

Compound D of Example 2 and Compound Ea of Example 8 were coupled under various reaction conditions to prepare F-3a corresponding to the compound represented by Formula 3.

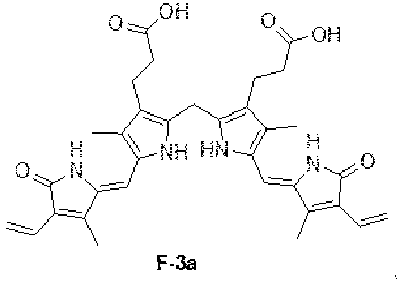

### Example 29: Preparation of F-3a

To a mixture of Compound D (1.29 g, 3.44 mmol, 1.0 equiv.) in 1,4-dioxane (20 mL), piperidine (2.35 g, 27.5 mmol, 8.0 equiv.) and Compound Ea (1.06 g, 8.61 mmol, 2.5 equiv.) were added and stirred at 100 °C for 14 hours under nitrogen condition. After removing the solvent under reduced pressure condition, CHCl₃ (600 mL) was added to the residue, stirred for 0.5 hours, and washed with 0.2 M aqueous hydrochloric acid (150 mL x 2). The organic layer was separated, dried over anhydrous Na₂SO₄, filtered and concentrated under reduced pressure condition. The residue was triturated with methanol (50 mL) at 20 °C. The red solid was filtered and washed with methanol (10 mL x 2) to yield Compound F-3a as a red solid (1.20 g, 2.05 mmol, yield: 60%).

¹H NMR (400 MHz, DMSO-*d₆*) δ 10.52 (brs, 2H), 9.95 (brs, 2H), 6.58 (dd, *J* = 17.6, 11.6 Hz, 2H), 6.20 (dd, *J* = 17.6, 2.8 Hz, 2H), 6.09 (s, 2H), 5.29 (dd, *J* = 11.6, 2.8 Hz, 2H), 3.99 (s, 2H), 2.50 - 2.43 (m, 4H), 2.16 (s, 6H), 2.03 (s, 6H), 2.00 - 1.85 (m, 4H).

### Example 30: Preparation of F-3a

To a mixture of Compound D (1.0 equiv.) in 1,4-dioxane, pyrrolidine (8.0 equiv.) and Compound Ea (2.5 equiv.) were added and stirred at 100 °C for 16 hours under nitrogen condition. A reaction conversion ratio was measured by liquid chromatography mass spectrometry (LCMS), and the reaction conversion ratio calculated by standardization was 9%.

### Example 31: Preparation of F-3a

To a mixture of Compound D (1.0 equiv.) and 1,4-dioxane, piperazine (8.0 equiv.) and Compound Ea (2.5 equiv.) were added and stirred 100 °C for 16 hours under nitrogen condition. After removing the solvent under reduced pressure condition, CHCl₃ (600 mL) was added to the residue, stirred for 0.5 hours, and washed with 0.2 M aqueous hydrochloric acid (150 mL x 2). The organic layer was separated, dried over anhydrous Na₂SO₄, filtered and concentrated under reduced pressure condition. The residue was triturated with methanol (50 mL) at 20 °C. The red solid was filtered and washed with methanol (10 mL x 2) to yield Compound F-3a as a red solid (yield: 15%).

### Example 32: Preparation of F-3a

To a mixture of Compound D (1.0 equiv.) and 1,4-dioxane, morpholine (8.0 equiv.) and Compound Ea (2.5 equiv.) were added, and stirred at 100 °C for 16 hours under nitrogen condition. A reaction conversion ratio was measured by liquid chromatography mass spectrometry (LCMS), and the reaction conversion ratio calculated by standardization was 37%.

### Example 33: Preparation of F-3a

To a mixture of Compound D (1.0 equiv.) and 1,4-dioxane, piperidine (8.0 equiv.) and Compound Ea (2.5 equiv.) were added and stirred at 25 °C for 16 hours under nitrogen condition. A reaction conversion ratio was measured by liquid chromatography mass spectrometry (LCMS), and the reaction conversion ratio calculated by standardization was 20%.

### Example 34: Preparation of F-3a

To a mixture of Compound D (1.0 equiv.) and 1,4-dioxane, piperidine (8.0 equiv.) and Compound Ea (2.5 equiv.) were added, and stirred at 25 °C for 8 hours under nitrogen condition. A reaction conversion ratio was measured by liquid chromatography mass spectrometry (LCMS), and the reaction conversion ratio calculated by standardization was 11%.

### Example 35: Preparation of F-3a

To a mixture of Compound D (1.0 equiv.) and 1,4-dioxane, piperidine (8.0 equiv.) and Compound Ea (2.5 equiv.) were added and stirred for 8 hours under nitrogen conditions at 100 °C for 8 hours under nitrogen condition. A reaction conversion ratio was measured by liquid chromatography mass spectrometry (LCMS), and the reaction conversion ratio calculated by standardization was 38%.

### Example 36: Preparation of F-3a

To a mixture of Compound D (1.0 equiv.) and MeOH, piperidine (8.0 equiv.) and Compound Ea (2.5 equiv.) were added, and stirred at 25 °C for 16 hours under nitrogen condition. A reaction conversion ratio was measured by liquid chromatography mass spectrometry (LCMS), and the reaction conversion ratio calculated by standardization was 11%.

### Example 37: Preparation of F-3a

To a mixture of Compound D (1.0 equiv.) and MeOH, piperidine (8.0 equiv.) and Compound Ea (2.5 equiv.) were added and stirred at 25 °C for 8 hours under nitrogen condition. A reaction conversion ratio was measured by liquid chromatography mass spectrometry (LCMS), and the reaction conversion ratio calculated by standardization was 9%.

### Example 38: Preparation of F-3a

To a mixture of Compound D (1.0 equiv.) and MeOH, piperidine (8.0 equiv.) and Compound Ea (2.5 equiv.) were added and stirred at 65 °C for 8 hours under nitrogen condition. A reaction conversion ratio was measured by liquid chromatography mass spectrometry (LCMS), and the reaction conversion ratio calculated by standardization was 7%.

### Example 39: Preparation of F-3a

To a mixture of Compound D (1.0 equiv.) and MeOH, piperidine (8.0 equiv.) and Compound Ea (2.5 equiv.) were added and stirred at 66 °C for 16 hours under nitrogen condition. A reaction conversion ratio was measured by liquid chromatography mass spectrometry (LCMS), and the reaction conversion ratio calculated by standardization was 4%.

### Example 40: Preparation of F-3a

To a mixture of Compound D (1.0 equiv.) and THF, piperidine (8.0 equiv.) and Compound Ea (2.5 equiv.) were added and stirred at 25 °C for 16 hours under nitrogen condition. A reaction conversion ratio was measured by liquid chromatography mass spectrometry (LCMS), and the reaction conversion ratio calculated by standardization was 22%.

### Example 41: Preparation of F-3a

To a mixture of Compound D (1.0 equiv.) and THF, piperidine (8.0 equiv.) and Compound Ea (2.5 equiv.) were added and stirred at 25 °C for 8 hours under nitrogen condition. A reaction conversion ratio was measured by liquid chromatography mass spectrometry (LCMS), and the reaction conversion ratio calculated by standardization was 17%.

### Example 42: Preparation of F-3a

To a mixture of Compound D (1.0 equiv.) and THF, piperidine (8.0 equiv.) and Compound Ea (2.5 equiv.) were added and stirred at 66 °C for 8 hours under nitrogen condition. A reaction conversion ratio was measured by liquid chromatography mass spectrometry (LCMS), and the reaction conversion ratio calculated by standardization was 51%.

### Example 43: Preparation of F-3a

To a mixture of Compound D (1.0 equiv.) and THF, piperidine (8.0 equiv.) and Compound Ea (2.5 equiv.) were added and stirred at 66 °C for 16 hours under nitrogen condition. A reaction conversion ratio was measured by liquid chromatography mass spectrometry (LCMS), and the reaction conversion ratio calculated by standardization was 10%.

### Example 44: Preparation of F-3a

To a mixture of Compound D (1.0 equiv.) and 1,4-dioxane, azepane (8.0 equiv.) and Compound Ea (2.5 equiv.) were added and stirred at 60 °C for 22 hours under nitrogen condition. After removing the solvent under reduced pressure condition, CHCl₃ (600 mL) was added to the residue, stirred for 0.5 hours, and washed with 0.2 M aqueous hydrochloric acid (150 mL × 2). The organic layer was separated, dried over anhydrous Na₂SO₄, filtered and concentrated under reduced pressure condition. The residue was triturated with methanol (50 mL) at 20 °C. The red solid was filtered and washed with methanol (10 mL × 2) to yield Compound F-3a as a red solid (yield: 25%).

### Example 45: Preparation of F-3a

To a mixture of Compound D (1.0 equiv.) and 1,4-dioxane, azocane (8.0 equiv.) and Compound Ea (2.5 equiv.) were added and stirred at 100 °C for 16 hours under nitrogen condition. After removing the solvent under reduced pressure condition, CHCl₃ (600 mL) was added to the residue, stirred for 0.5 hours, and then washed with 0.2 M aqueous hydrochloric acid (150 mL × 2). The organic layer was separated, dried over anhydrous Na₂SO₄, filtered and concentrated under reduced pressure condition. The residue was triturated with methanol (50 mL) at 20 °C. The red solid was filtered and washed with methanol (10 mL × 2) to yield Compound F-3a as a red solid (yield: 26%).

### 3.2. Preparation of Compound F-3a by coupling of Compound D and Compound Ea-3a

F-3a corresponding to the compound represented by Formula 3 was prepared by coupling the Compound D of Example 2 with the Compound Ea-3a of Example 9 under various reaction conditions.

### Example 46: Preparation of F-3a

To a mixture of Compound D (50 mg, 0.13 mmol, 1.0 equiv.) in 1,4-dioxane (2 mL), piperidine (90.97 mg, 1.07 mmol, 8.0 equiv.) and Compound Ea-3a (56.56 mg, 0.40 mmol, 3.0 equiv.) were added and stirred at 100 °C for 16 hours under nitrogen condition. After concentrating under reduced pressure, CHCl₃ (110 mL×2) was added and washed with 0.3 M HCl aqueous solution (20 mL). The combined organic layer was dried over anhydrous Na₂SO₄, filtered under reduced pressure and concentrated. The residue was triturated with MeOH (6 mL) to yield Compound F-3a as a brown solid (18.1 mg, 30.9 µmol, yield: 23%).

### Example 47: Preparation of F-3a

To a mixture of Compound D (1.0 equiv.) in 1,4-dioxane, piperidine (8.0 equiv.) and Compound Ea-3a (3.0 equiv.) were added and stirred at 100 °C for 8 hours under nitrogen condition. A reaction conversion ratio was measured by liquid chromatography mass spectrometry (LCMS), and the reaction conversion ratio calculated by standardization was 15%.

### Example 48: Preparation of F-3a

To a mixture of Compound D (1.0 equiv.) in 1,4-dioxane, pyrrolidine (8.0 equiv.) and Compound Ea-3a (3.0 equiv.) were added and stirred at 100 °C for 8 hours under nitrogen condition. A reaction conversion ratio was measured by liquid chromatography mass spectrometry (LCMS), and the reaction conversion ratio calculated by standardization was 12%.

### Example 49: Preparation of F-3a

To a mixture of Compound D (1.0 equiv.) in 1,4-dioxane, pyrrolidine (8.0 equiv.) and Compound Ea-3a (3.0 equiv.) were added and stirred at 100 °C for 16 hours under nitrogen condition. A reaction conversion ratio was measured by liquid chromatography mass spectrometry (LCMS), and the reaction conversion ratio calculated by standardization was 11%.

### Example 50: Preparation of F-3a

To a mixture of Compound D (1.0 equiv.) in 1,4-dioxane, morpholine (8.0 equiv.) and Compound Ea-3a (3.0 equiv.) were added and stirred at 100 °C for 8 hours under nitrogen condition. After concentrating under reduced pressure, CHCl₃ (110 mL×2) was added and washed with 0.3 M HCl aqueous solution (20 mL). The combined organic layer was dried over anhydrous Na₂SO₄, filtered under reduced pressure and concentrated. The residue was triturated with MeOH to yield Compound F-3a as a brown solid (yield: 13%).

### Example 51: Preparation of F-3a

To a mixture of Compound D (1.0 equiv.) in 1,4-dioxane, morpholine (8.0 equiv.) and Compound Ea-3a (3.0 equiv.) were added and stirred at 100 °C for 16 hours under nitrogen condition. After concentrating under reduced pressure, CHCl₃ (110 mL×2) was added and washed with 0.3 M HCl aqueous solution (20 mL). The combined organic layer was dried over anhydrous Na₂SO₄, filtered under reduced pressure and concentrated. The residue was triturated with MeOH to yield Compound F-3a as a brown solid (yield: 10%).

### Example 52: Preparation of F-3a

To a mixture of Compound D (1.0 equiv.) in 1,4-dioxane, piperazine (8.0 equiv.) and Compound Ea-3a (3.0 equiv.) were added and stirred at 100 °C for 8 hours under nitrogen condition. After concentrating under reduced pressure, CHCl₃ (110 mL×2) was added and washed with 0.3 M HCl aqueous solution (20 mL). The combined organic layer was dried over anhydrous Na₂SO₄, filtered under reduced pressure and concentrated. The residue was triturated with MeOH to yield Compound F-3a as a brown solid (yield: 18%).

### Example 53: Preparation of F-3a

To a mixture of Compound D (1.0 equiv.) in 1,4-dioxane, piperazine (8.0 equiv.) and Compound Ea-3a (3.0 equiv.) were added and stirred at 100 °C for 16 hours under nitrogen condition. After concentrating under reduced pressure, CHCl₃ (110 mL×2) was added and washed with 0.3 M HCl aqueous solution (20 mL). The combined organic layer was dried over anhydrous Na₂SO₄, filtered under reduced pressure and concentrated. The residue was triturated with MeOH to yield Compound F-3a as a brown solid (yield: 17%).

### 3.3. Preparation of Compound F-3a by coupling of Compound D and Compound Ea-3c

F-3a corresponding to the compound represented by Formula 3 was prepared by coupling the Compound D of Example 2 with the Compound Ea-3c of Example 15 under various reaction conditions.

### Example 54: Preparation of F-3a

To a mixture of Compound D (30 mg, 80.13 µmol, 1.0 equiv.) and Compound Ea-3c (47.89 mg, 0.24 mmol, 3.0 equiv.) in 1,4-dioxane (3 mL), piperidine (54.58 mg, 0.64 mmol, 8.0 equiv.) was added and stirred at 100 °C for 16 hours. After concentrating under reduced pressure, CHCl₃ (110 mL×2) was added and washed with 0.3 M HCl aqueous solution (20 mL). The combined organic layer was dried over anhydrous Na₂SO₄, filtered under reduced pressure and concentrated, followed by confirming F-3a through LCMS.
C₃₃H₃₆N₄O₆ m/z [M+H]⁺ = 585

### 3.4. Preparation of Compound D-Ea-2a by coupling of Compound D and Compound Ea-2a

D-Ea-2a corresponding to the compound represented by Formula 3 was prepared by coupling Compound D of Example 2 with Compound Ea-2a of Example 10, and Compound F was prepared therefrom.

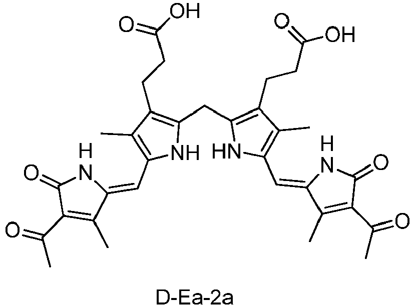

### Example 55: Preparation of Compound D-Fa-2a

A mixture of Compound D (64 mg, 0.17 mmol, 1.0 equiv.) and azepane (0.11 mL, 1.02 mmol, 6.0 equiv.) in 1,4-dioxane (3.6 mL) was stirred at 25 °C for 10 minutes. Compound Ea-2a (60 mg, 0.43 mmol, 2.5 equiv.) was added to the mixture and stirred at 40 °C for 16 hours. CHCl₃ (100 mL) was added and the organic layer was washed with 0.2 M HCl aqueous solution (80 mL × 2). The combined organic layer was dried over anhydrous Na₂SO₄, filtered and concentrated under reduced pressure condition. After dissolving the residue in EtOAc (5 mL) and stirring for 5 minutes, hexane (30 mL) was added dropwise thereto. At this time, the precipitated red solid was filtered to yield Compound D-Ea-2a (100 mg, 0.16 mmol, yield: 94%).

¹H NMR (400 MHz, DMSO-*d₆*) δ 11.94 (brs, 2H), 10.84 (s, 2H), 10.14 (s, 2H), 6.45 (s, 2H), 4.08 (s, 2H), 2.46 (s, 6H), 2.45 (s, 6H), 2.45 - 2.40 (m, 4H), 2.09 (s, 6H), 1.95 - 1.85 (m, 4H).

### Example 56: Preparation of Compound F-3a from Compound D-Ea-2a

### (56-1) Preparation of Compound D-Ea-3a

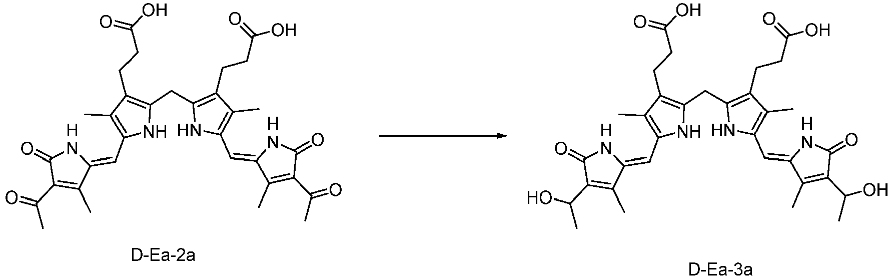

To a mixture of Compound D-Ea-2a (100 mg, 0.16 mmol, 1.0 equiv.) prepared above in DMSO (6 mL), NaBH₄ (24.2 mg, 0.64 mmol, 4.0 equiv.) was added and stirred at 25 °C for 16 hours. After the reaction was terminated with NH₄Cl aqueous solution (50 mL), the mixture was extracted with chloroform (100 mL × 3) and washed with brine (50 mL × 5). The combined organic layer was dried over anhydrous Na₂SO₄, filtered and concentrated under reduced pressure condition to yield Compound D-Ea-3a (20 mg, 0.03 mmol, yield: 20%).

¹HNMR(400 MHz, DMSO-*d₆*) δ 11.87 (brs, 2H), 10.35 (brs, 2H), 9.77 (s, 2H), 5.98 (s, 2H), 4.67 - 4.65 (m, 2H), 3.96 (s, 2H), 2.43 - 2.41 (m, 4H), 2.29 (s, 6H), 2.00 (s, 6H), 1.97 - 1.94 (m, 4H), 1.31 (d, *J* = 6.8 Hz, 6H).

### (56-2) Preparation of Compound F-3a

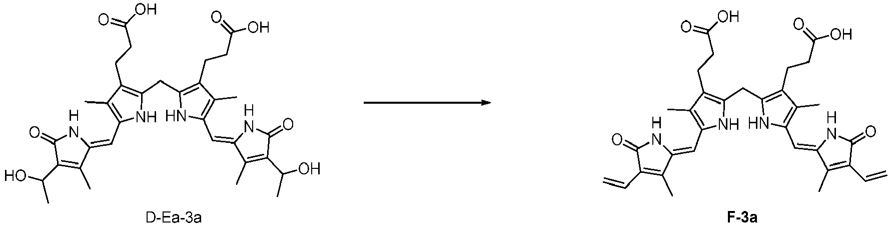

To a mixture of Compound D-Ea-3a (10 mg, impure) prepared above and DCM (1 mL), TEA (27 µL, 0.19 mmol, 12.0 equiv.) was added, followed by adding a mixture of POCl₃ (6.02 µL. 0.064 mmol, 4.0 equiv.) in DCM (30 µL) to the above mixture at 0 °C. The mixture was stirred at 25 °C for 3 hours under nitrogen condition. The reaction was terminated by adding water (30 mL), followed by extraction with CHCl₃ (50 mL × 2). The extracted solution was dried over anhydrous Na₂SO₄, filtered and concentrated under reduced pressure condition. The residue was triturated with a DCM:MeOH (1: 1) solution and filtered to yield Compound F-3a as an orange solid.

### 3.5. Preparation of Compound D-Eb by coupling of Compound D and Compound Eb

D-Eb corresponding to the compound represented by Formula 3 was prepared by coupling Compound D of Example 2 with Compound Eb of Example 24, and Compound F-3a was prepared therefrom.

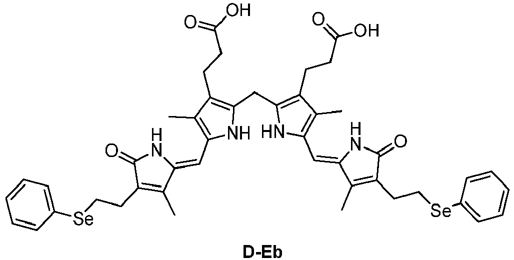

### Example 57: Preparation of Compound D-Eb

To a mixture of Compound D (268 mg, 0.71 mmol, 1.0 equiv.) and piperidine (10.0 equiv.) in 1,4-dioxane (15 mL), Compound Eb (501 mg, 1.80 mmol, 2.5 equiv.) was added, followed by stirring the mixture at 100 °C for 16 hours. 0.1 M HCl aqueous solution (71.6 mL) was added to the mixture, followed by extraction with EtOAc (50 mL × 3). The combined organic layer was dried over anhydrous Na₂SO₄, filtered and concentrated under reduced pressure condition. The residue was triturated with MeOH (20 mL) at 20 °C. The resulting precipitate was collected by filtration and washed with MeOH (10 mL × 2) to yield Compound D-Eb as a yellow solid (471 mg, 0.52 mmol, yield: 73%).

¹H NMR (400 MHz, DMSO-*d₆*) δ 11.91 (brs, 2H), 10.36 (s, 2H), 9.82 (s, 2H), 7.50 - 7.48 (m, 4H), 7.31 - 7.23 (m, 6H), 5.97 (s, 2H), 3.96 (s, 2H), 3.09 (t, *J* = 7.6 Hz, 4H), 2.63 (t, *J* = 7.6 Hz, 4H), 2.41 (t, *J* = 7.2 Hz, 4H), 2.01 (s, 6H), 2.00 (s, 6H), 1.96 (t, *J* = 8.0 Hz, 4H).

### Example 58: Preparation of Compound F-3a from Compound D-Eb

To a mixture of Compound D-Eb (100 mg, 0.11 mmol, 1.0 equiv.) dissolved in THF (8 mL), HOAc (20 mg, 0.33 mmol, 3.0 equiv.) and H₂O₂ (51 mg, 0.45 mmol, 4.0 equiv.) were added, followed by stirring the mixture at 25 °C for 1 hour. After concentrating the mixture under reduced pressure, the residue was triturated with MeOH (10 mL) at 20 °C. The precipitate was collected by filtration and washed with MeOH (5 mL × 2) to yield F-3a corresponding to the compound represented by Formula 3 herein as a red solid (10 mg, 0.017 mmol, yield: 15%).

¹H NMR (400 MHz, DMSO-*d₆*) δ 11.74 (brs, 2H), 10.51 (s, 2H), 9.95 (s, 2H), 6.58 (dd, *J* = 17.6, 11.6 Hz, 2H), 6.20 (dd, *J* = 17.6, 2.8 Hz, 2H), 6.08 (s, 2H), 5.29 (dd, *J* = 11.6, 2.8 Hz, 2H), 3.98 (s, 2H), 2.43 (t, *J=* 8.4 Hz, 4H), 2.16 (s, 6H), 2.03 (s, 6H), 1.94 (t, *J=* 8.4 Hz, 4H).

### 3.6. Preparation of Compound D-Ed by coupling of Compound D and Compound Ed

D-Ed corresponding to the compound represented by Formula 3 was prepared by coupling the Compound D of Example 2 with the Compound Ed of Example 26, and Compound F-3a was prepared therefrom.

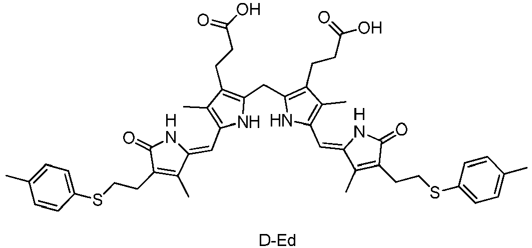

### Example 59: Preparation of Compound D-Ed

A mixture of Compound D (16.3 mg, 0.04 mmol, 1.0 equiv.) and azepane (29.8 uL, 0.26 mmol, 6.0 equiv.) in 1,4-dioxane (5 mL) was stirred at 25 °C for 10 minutes. The Compound Ed (21.6 mg, 0.09 mmol, 2 equiv.) prepared above was added to the above mixture, and stirred at 80 °C for 16 hours. 0.1 M HCl aqueous solution (10 mL) was added to the mixture, and extracted with CHCl₃ (10 mL × 3). The combined organic layer was dried over anhydrous Na₂SO₄, filtered and concentrated under reduced pressure to yield Compound D-Ed.
C₄₇H₅₂N₄O₆S₂ m/z [M+2H]⁺ = 834

### Example 60: Preparation of Compound F-3a from Compound D-Ed

A mixture of D-Ed (30 mg) prepared above in 1,4-dioxane (8 mL) was cooled to 0 °C, followed by addition of m-CPBA (9 mg, 0.04 mmol). Then, the mixture was stirred at 0 °C for 1 hour. An aqueous solution of NaHSO₃ was added to the mixture, followed by extraction with CHCl₃, and the combined organic layer was dried with anhydrous Na₂SO₄, filtered and concentrated under reduced pressure condition. After dissolving the residue in DMF (8 mL), pyridine (3 mL) was added thereto, followed by refluxing for 2 hours. The mixture was layer-separated with 0.1 M HCl and CHCl₃, and the organic layer was dried over anhydrous Na₂SO₄, filtered and concentrated under reduced pressure condition, followed by confirming Compound F-3a through LCMS.
C₃₃H₃₆N₄O₆ m/z [M+2H]⁺ = 586

### 3.7. Preparation of Compound D-Ee by coupling of Compound D and Compound Ee

D-Ee corresponding to the compound represented by Formula 3 was prepared by coupling the Compound D of Example 2 with the Compound Ee of Example 28.

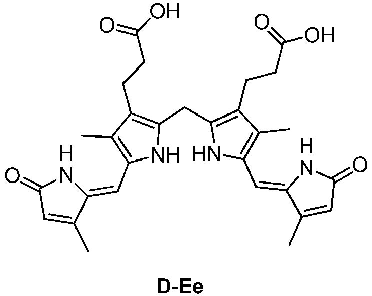

### Example 61: Preparation of Compound D-Ee by coupling Compound D and Compound Ee

To a mixture of Compound D (100 mg, 0.26 mmol, 1.0 equiv.) in 1,4-dioxane (9 mL), azepane (0.18 mL, 1.60 mmol, 6.0 equiv.) was added and stirred at 25 °C for 10 minutes. Compound Ee (64.8 mg, 0.67 mmol, 2.5 equiv.) was added to the above mixture and stirred at 40 °C for 15 hours. CHCl₃ (100 mL) was added to the mixture and washed with 0.1 M hydrochloric acid aqueous solution (30 mL × 2). The combined organic layer was dried over anhydrous Na₂SO₄, filtered and concentrated under reduced pressure condition. The obtained residue was purified by silica gel chromatography to yield Compound D-Ee as a green solid (32 mg, 0.057 mmol, yield: 22%).

¹H NMR (400 MHz, DMSO-*d₆*) δ 11.90 (brs, 2H), 10.40 (s, 2H), 9.77 (s, 2H), 5.99 (s, 2H), 5.73 (s, 2H), 3.97 (s, 2H), 2.41 (t, *J=* 6.0 Hz, 4H), 2.14 (s, 6H), 2.01 (s, 6H), 1.95 (t, *J=* 6.1 Hz, 4H).

### 3.8. Preparation of Compound C-Ea by coupling of Compound C and Compound Ea

Compound C-Ea corresponding to the compound represented by Formula 3 (Examples 62 to 67) was prepared by coupling Compound C of Example 1 with Compound Ea of Example 8 under various reaction conditions, and Compound F-3a was prepared therefrom (Example 68).

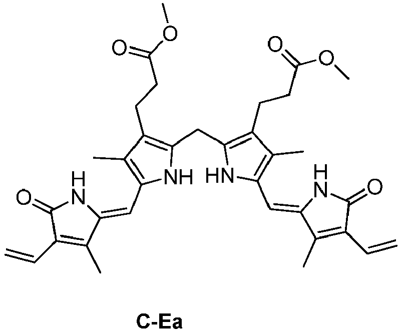

### Example 62: Preparation of Compound C-Ea

To a mixture of Compound C (1.09 g, 2.71 mmol, 1.0 equiv.) and Compound Ea (1.00 g, 8.12 mmol, 3.0 equiv.) in 1,4-dioxane (10 mL), piperidine (1.84 g, 21.7 mmol, 8.0 equiv.) was added and stirred at 80 °C for 12 hours. The reactant was concentrated under reduced pressure condition and purified by prep-HPLC. The remaining solvent was removed by lyophilization to yield Compound C-Ea as a black solid (10 mg, 16.3 µmol, yield: 0.6%).

¹H NMR (400 MHz, DMSO-*d₆*) δ 10.62 (s, 2H), 9.96 (s, 2H), 6.65 (dd, *J =* 17.2, 11.2 Hz, 2H), 6.28 (dd, *J=* 17.2, 2.4 Hz, 2H), 6.15 (s, 2H), 5.37 (d, *J =* 12.0 Hz, 2H), 4.05 (s, 2H), 3.49 (s, 6H), 2.51 - 2.41 (m, 4H), 2.23 (s, 6H), 2.08 (s, 6H), 1.98 - 1.94 (m, 4H).

### Example 63: Preparation of Compound C-Ea

To a mixture of Compound C (1.0 equiv.) and Compound Ea (3.0 equiv.) in 1,4-dioxane, piperazine (8.0 equiv.) was added and stirred at 100 °C for 8 hours. A reaction conversion ratio was measured by liquid chromatography mass spectrometry (LCMS), and the reaction conversion ratio calculated by standardization was 27%.

### Example 64: Preparation of Compound C-Ea

To a mixture of Compound C (1.0 equiv.) and Compound Ea (3.0 equiv.) in 1,4-dioxane, piperazine (8.0 equiv.) was added and stirred at 100 °C for 16 hours. A reaction conversion ratio was measured by liquid chromatography mass spectrometry (LCMS), and the reaction conversion ratio calculated by standardization was 21%.

### Example 65: Preparation of Compound C-Ea

To a mixture of Compound C (1.0 equiv.) and Compound Ea (3.0 equiv.) in 1,4-dioxane, morpholine (8.0 equiv.) was added and stirred at 100 °C for 8 hours. A reaction conversion ratio was measured by liquid chromatography mass spectrometry (LCMS), and the reaction conversion ratio calculated by standardization was 35%.

### Example 66: Preparation of Compound C-Ea

To a mixture of Compound C (1.0 equiv.) and Compound Ea (3.0 equiv.) in 1,4-dioxane, morpholine (8.0 equiv.) was added and stirred at 100 °C for 16 hours. A reaction conversion ratio was measured by liquid chromatography mass spectrometry (LCMS), and the reaction conversion ratio calculated by standardization was 38%.

### Example 67: Preparation of Compound C-Ea

To a mixture of Compound C (1.0 equiv.) and Compound Ea (3.0 equiv.) in 1,4-dioxane, proline (8.0 equiv.) was added and stirred at 100 °C for 16 hours. The reactant was concentrated under reduced pressure condition and purified by prep-HPLC. The remaining solvent was removed through lyophilization to yield Compound C-Ea as a black solid (yield: 0.9%).

### Example 68: Preparation of Compound F-3a from Compound C-Ea

To a solution of Compound C-Ea (50 mg, 81.60 µmol, 1.0 equiv.) prepared above in methanol (5 mL), a mixture of LiOH·H₂O (20.55 mg, 0.49 mmol, 6.0 equiv.) in water (1 mL) was added and stirred at 60 °C for 2 hours under nitrogen condition. CHCl₃ (20 mL) was added to the mixture, and the pH was adjusted acidic with 0.1 M HCl aqueous solution (10 mL). The combined organic layer was dried over anhydrous Na₂SO₄, filtered and concentrated under reduced pressure condition. Methanol (10 mL) was added to the residue, and the precipitated solid was filtered under reduced pressure to yield Compound F-3a (bilirubin) as an orange solid (10 mg, 17.10 µmol, yield: 21%).

¹H NMR (400 MHz, DMSO-*d₆*) δ 10.50 (s, 2H), 9.93 (s, 2H), 6.58 (dd, *J =* 17.2, 11.2 Hz, 2H), 6.21 (dd, *J* = 17.6, 2.4 Hz, 2H), 6.07 (s, 2H), 5.30 (dd, *J =* 11.6, 2.8 Hz, 2H), 3.99 (s, 2H), 2.42 - 2.30 (m, 4H), 2.16 (s, 6H), 2.03 (s, 6H), 1.95 - 1.91 (m, 4H).

### 3.9. Preparation of Compound C-Ea by coupling of Compound C and Compound Ea-3a

Compound C-Ea corresponding to the compound represented by Formula 3 (Examples 69 to 72) was prepared by coupling Compound C of Example 1 with Compound Ea-3a of Example 9 under various reaction conditions.

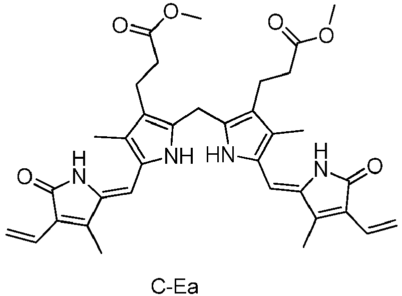

### Example 69: Preparation of Compound C-Ea

To a mixture of Compound C (1.33 g, 3.31 mmol, 1.0 equiv.) in 1,4-dioxane (20 mL), piperidine (2.81 g, 33.1 mmol, 3.26 mL, 10.0 equiv.) and Compound Ea-3a (1.4 g, 9.92 mmol, 3.0 equiv.) were added and stirred at 101 °C for 16 hours under nitrogen condition. CHCl₃ (350 mL) was added to the mixture and washed with 0.1 M HCl aqueous solution (150 mL × 2). The combined organic layer was washed with brine (50 mL), dried over anhydrous Na₂SO₄, filtered and concentrated under reduced pressure condition. The residue was purified by prep-HPLC. The solvent was removed through lyophilization to yield Compound C-Ea as a black solid (415 mg, 0.68 mmol, yield: 20%).

¹HNMR (400 MHz, DMSO-*d₆*) δ 10.55 (s, 2H), 9.88 (s, 2H), 6.58 (dd, *J=* 17.2, 11.6 Hz, 2H), 6.21 (dd, *J* = 17.2, 2.8 Hz, 2H), 6.07 (s, 2H), 5.30 (dd, *J* = 11.6, 2.4 Hz, 2H), 3.98 (s, 2H), 3.28 (s, overlapped H₂O peak 6H), 2.50 (t, overlap with DMSO-*d₆*'s signal, 4H), 2.16 (s, 6H), 2.01 (s, 6H), 1.88 (t, *J=* 8.4 Hz, 4H).

### Example 70: Preparation of Compound C-Ea

To a mixture of Compound C (1.0 equiv.) in 1,4-dioxane, pyrrolidine (10.0 equiv.) and Compound Ea-3a (3.0 equiv.) were added and stirred at 101 °C for 16 hours under nitrogen condition. A reaction conversion ratio was measured by liquid chromatography mass spectrometry (LCMS), and the reaction conversion ratio calculated by standardization was 23%.

### Example 71: Preparation of Compound C-Ea

To a mixture of Compound C (1.0 equiv.) in 1,4-dioxane, piperazine (10.0 equiv.) and Compound Ea-3a (3.0 equiv.) were added and stirred at 101 °C for 16 hours under nitrogen condition. A reaction conversion was measured by liquid chromatography mass spectrometry (LCMS), and the reaction conversion ratio calculated by standardization was 37%.

### Example 72: Preparation of Compound C-Ea

To a mixture of Compound C (1.0 equiv.) in 1,4-dioxane, morpholine (10.0 equiv.) and Compound Ea-3a (3.0 equiv.) were added and stirred at 101 °C for 16 hours under nitrogen condition. A reaction conversion ratio was measured by liquid chromatography mass spectrometry (LCMS), and the reaction conversion ratio calculated by standardization was 32%.

### 3.10. Preparation of Compound C1-Ea by coupling of Compound C1 and Compound Ea

C1-Ea corresponding to the compound represented by Formula 3 was prepared by coupling Compound C1 of Example 3 with Compound Ea of Example 8, and Compound F-3a was prepared therefrom.

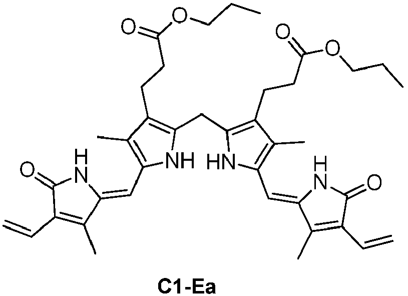

### Example 73: Preparation of Compound C1-Ea

To a mixture of Compound C1 (80 mg, 0.17 mmol, 1.0 equiv.) in 1,4-dioxane (1 mL), piperidine (0.14 g, 1.7 mmol, 10.0 equiv.) and Compound Ea (62.8 mg, 0.51 mmol, 3.0 equiv.) were added and stirred at 101 °C for 16 hours under nitrogen condition. CHCl₃ (30 mL) was added to the mixture and washed with 0.1 M HCl aqueous solution (20 mL × 2). The combined organic layer was washed with brine (20 mL), dried over anhydrous Na₂SO₄, filtered and concentrated under reduced pressure condition. DCM/Hexanes were added dropwise to the residue, and the obtained solid was filtered to yield Compound C1-Ea as a red solid (40 mg, 0.059 mmol, yield: 35%).

¹H NMR (400 MHz, DMSO-*d₆*) δ 10.58 (brs, 2H), 9.89 (brs, 2H), 6.58 (dd, *J* = 17.2, 11.6 Hz, 2H), 6.21 (2H, d, *J* = 17.2 Hz), 6.06 (2H, s), 5.30 (d, *J* = 11.6 Hz, 2H), 3.98 (s, 2H), 3.77 (t, *J=* 6.0 Hz, 4H), 2.50 - 2.40 (m, overlap with DMSO-*d₆*'s signal, 4H), 2.15 (s, 6H), 2.01 (s, 6H), 1.89 - 1.77 (m, 4H), 1.45 - 1.40 (m, 4H), 0.76 (t, *J=* 7.2 Hz, 6H).

### Example 74: Preparation of Compound F-3a from Compound C1-Ea

To a mixture of Compound C1-Ea (50 mg, 0.074 mmol, 1.0 equiv.) prepared above in methanol (1 mL), a mixture of LiOH·H₂O (18.45 mg, 0.44 mmol, 6.0 equiv.) in water (1 mL) was added and stirred at 60 °C for 3 hours under nitrogen condition. CHCl₃ (20 mL) was added to the mixture and the pH was adjusted acidic with 0.1 M HCl aqueous solution (10 mL). The combined organic layer was dried over anhydrous Na₂SO₄, filtered and concentrated under reduced pressure condition. DCM/Hexanes were added to the residue, and the resulting solid was filtered under reduced pressure to yield Compound F-3a (bilirubin) as an orange solid (15 mg, 0.025 mmol, yield: 34%).

### 3.11. Preparation of Compound C2-Ea by coupling of Compound C2 and Compound Ea

Compound C2-Ea corresponding to the compound represented by Formula 3 was prepared by coupling Compound C2 of Example 4 with Compound Ea of Example 8, and Compound F-3a was prepared therefrom.

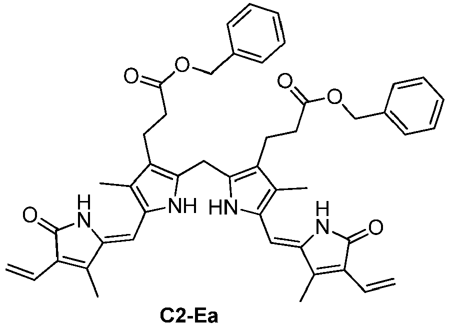

### Example 75: Preparation of Compound C2-Ea

To a mixture of Compound C2 (90 mg, 0.16 mmol, 1.0 equiv.) in 1,4-dioxane (1 mL), piperidine (0.13 g, 1.6 mmol, 10.0 equiv.) and Compound Ea (59.1 mg, 0.48 mmol, 3.0 equiv.) prepared above were added and stirred at 101 °C for 16 hours under nitrogen condition. CHCl₃ (30 mL) was added to the mixture and washed with 0.1 M HCl aqueous solution (20 mL × 2). The combined organic layer was washed with brine (20 mL), dried over anhydrous Na₂SO₄, filtered and concentrated under reduced pressure condition. The residue was solidified via EtOAc/Hexanes to yield Compound C2-Ea as a dark orange solid (45 mg, 0.058 mmol, yield: 36%).

¹H NMR (400 MHz, DMSO-*d₆*) δ 10.59 (brs, 2H), 9.92 (brs, 2H), 7.32-7.02 (m, 10H), 6.58 (dd, *J* = 17.6 Hz, *J* = 11.6 Hz, 2H), 6.22 (dd, *J* = 17.2, 2.4 Hz, 2H), 6.06 (s, 2H), 5.31 (dd, *J* = 12.0, 2.0 Hz, 2H), 4.92 (s, 4H), 4.00 (s, 2H), 2.50 - 2.40 (m, overlap with DMSO-*d₆*'s signal, 4H), 2.10 (s, 6H), 2.02-1.97 (m, 10H).

### Example 76: Preparation of Compound F-3a from Compound C2-Ea

To a mixture of Compound C2-Ea (60 mg, 0.078 mmol, 1.0 equiv.) prepared above in THF (2 mL), Pd/C (5.0 mg, 10 mol%) was added under nitrogen condition. The mixture was degassed under vacuum condition and filled with H₂ several times. The mixture was stirred at 25 °C for 6 hours under H₂ (15 psi) condition, followed by confirming production of Compound F-3a through LCMS.
C₃₃H₃₆N₄O₆ m/z [M+H]⁺ = 585

### 4. PEGylation of compound represented by Formula 3

F-3a corresponding to the compound represented by Formula 3 was PEGylated under various reaction conditions.

### Example 77: Preparation of FP-3a (mono-PEGylation)

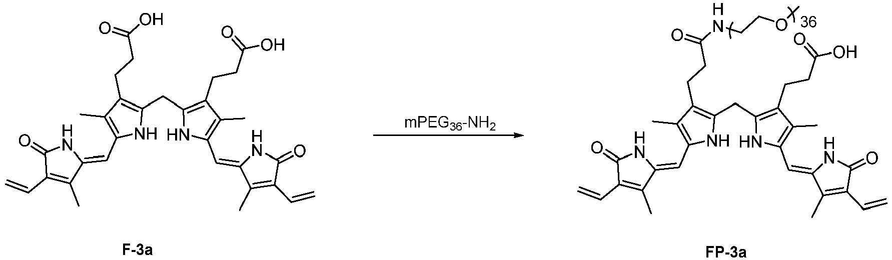

A mixture of Compound F-3a (440 mg, 0.75 mmol, 1.0 equiv.) in DMSO (22 mL) was stirred at 25 °C for 15 minutes. A solution of CDI (183 mg, 1.13 mmol, 1.5 equiv.) in DMSO (8.8 mL) was added dropwise to the above mixture. After stirring at 25 °C for 2 hours, a mixture of mPEG₃₆-NH₂ (487 mg, 0.30 mmol, 0.4 equiv.) in DMSO (4.4 mL) was added and stirred at 25 °C for 4 hours. After adding Na₂CO₃ aqueous solution (220 mL) to the mixture, it was stirred until it became a clear yellow solution. The solution was extracted with chloroform (200 mL × 3) and the combined organic layer was dried over anhydrous MgSO₄, filtered and concentrated under reduced pressure condition. The residue was purified by column chromatography to yield Compound FP-3a (220 mg, 0.25 mmol, yield: 33%), which was mono-PEGylated from the compound corresponding to the compound represented by Formula 3 herein, as an orange solid.

¹H NMR(400 MHz, DMSO-*d₆*) δ 11.91 (s, 1H), 10.50-10.40 (m, 2H), 9.92 (s, 2H), 7.62 - 7.60 (m 1H), 6.58 (dd, *J=* 14.0, 10.0 Hz, 2H), 6.21 (d, *J =* 14.0 Hz, 2H), 6.09 (s, 2H), 5.29 (d, *J* = 9.2 Hz, 2H), 3.98 (s, 2H), 3.65 - 3.41 (m, 142H), 3.24 (s, 3H), 3.13 - 3.12 (m, 2H), 2.47 - 2.40 (m, 4H), 2.16 (s, 6H), 2.04 (s, 6H), 1.96 - 1.93 (m, 4H).

### Example 78: Preparation of FP-3a (monoPEGvlation)

A mixture of Compound F-3a (1.0 equiv.) in DMSO was stirred at 25 °C for 15 minutes. A solution of CDI (1.5 equiv.) in DMSO was added dropwise to this mixture. After stirring at 25 °C for 2 hours, a mixture of mPEG₃₆-NH₂ (0.4 equiv.) in DMSO was added and stirred at 25 °C for 16 hours. A reaction conversion ratio was measured by liquid chromatography mass spectrometry (LCMS), and the reaction conversion ratio calculated by standardization was 30%.

### Example 79: Preparation of FP-3a (monoPEGvlation)

A mixture of Compound F-3a (1.0 equiv.) in DMSO/DMF was stirred at 25 °C for 15 minutes. Then, a solution of EDCI (1.5 equiv.) in DMSO/DMF was added dropwise to the above mixture. After stirring at 25 °C for 2 hours, a mixture of mPEG₃₆-NH₂ (0.4 equiv.) in DMSO/DMF and pyridine (3.0 equiv.) were added and stirred at 25 °C for 4.5 hours. A reaction conversion ratio was measured by liquid chromatography mass spectrometry (LCMS), and the reaction conversion ratio calculated by standardization was 62%.

### Example 80: Preparation of FP-3a (monoPEGylation)

A mixture of Compound F-3a (1.0 equiv.) in DMSO was stirred at 25 °C for 15 minutes. A solution of HATU (1.2 eq.) in DMSO was added dropwise to the above mixture. After stirring at 25 °C for 2 hours, a mixture of mPEG₃₆-NH₂ (0.4 equiv.) in DMSO and DIPEA (3.0 equiv.) were added and stirred at 20 °C for 12 hours. A reaction conversion ratio was measured by liquid chromatography mass spectrometry (LCMS), and the reaction conversion ratio calculated by standardization was 10%.

### Example 81: Preparation of FP-3a (monoPEGylation)

A mixture of Compound F-3a (1.0 equiv.) in DMSO was stirred at 25 °C for 15 minutes. A solution of CMPI (0.9 equiv.) in DMSO was added dropwise to the above mixture. After stirring at 25 °C for 2 hours, a mixture of mPEG₃₆-NH₂ (0.4 equiv.) in DMSO and DIPEA (3.0 equiv.) were added and stirred at 25 °C for 1 hour. A reaction conversion ratio was measured by liquid chromatography mass spectrometry (LCMS), and the reaction conversion ratio calculated by standardization was 45%.

### Example 82: Preparation of FP-3a (monoPEGylation)

A mixture of Compound F-3a (1.0 equiv.) in DMSO/DMF was stirred at 25 °C for 15 minutes. A solution of EDCI (1.5 equiv.) in DMSO/DMF was added dropwise to the above mixture. After stirring at 25 °C for 2 hours, a mixture of mPEG₃₆-NH₂ (0.4 equiv.) in DMSO/DMF and pyridine (3.0 equiv.) were added and stirred at 20 °C for 12 hours. A reaction conversion ratio was measured by liquid chromatography mass spectrometry (LCMS), and the reaction conversion ratio calculated by standardization was 56%.

### Example 83: Preparation of FP-3a (monoPEGylation)

A mixture of Compound F-3a (1.0 equiv.) in DMF was stirred at 25 °C for 15 minutes. A solution of CDI (1.6 equiv.) in DMF was added dropwise to the above mixture. After stirring at 25 °C for 2 hours, a mixture of mPEG₃₆-NH₂ (0.4 equiv.) in DMF was added and stirred at 25 °C for 6.5 hours. A reaction conversion ratio was measured by liquid chromatography mass spectrometry (LCMS), and the reaction conversion ratio calculated by standardization was 12%.

### Example 84: Preparation of FP-3a (monoPEGylation)

A mixture of Compound F-3a (1.0 equiv.) in pyridine was stirred at 25 °C for 15 minutes. A solution of EDCI (0.8 equiv.) in pyridine was added dropwise to the above mixture. After stirring at 25 °C for 2 hours, a mixture of mPEG₃₆-NH₂ (0.4 equiv.) in pyridine was added and stirred at 0 °C for 2 hours. A reaction conversion ratio was measured by liquid chromatography mass spectrometry (LCMS), and the reaction conversion ratio calculated by standardization was 9%.

### Example 85: Preparation of FP-3a (monoPEGylation)

A mixture of Compound F-3a (1.0 equiv.) in pyridine was stirred at 25 °C for 15 minutes. A solution of EDCI (1.1 equiv.) in pyridine was added dropwise to the above mixture. After stirring at 25 °C for 2 hours, a mixture of mPEG₃₆-NH₂ (0.4 equiv.) in pyridine was added and stirred at 25 °C for 6.5 hours. A reaction conversion ratio was measured by liquid chromatography mass spectrometry (LCMS), and the reaction conversion ratio calculated by standardization was 18%.

### Example 86: Preparation of FP-3a (monoPEGylation)

A mixture of Compound F-3a (1.0 equiv.) in pyridine was stirred at 25 °C for 15 minutes. A solution of EDCI (2.0 equiv.) and HOBt (2.2 equiv.) in DMF was added dropwise to the above mixture. After stirring at 25 °C for 2 hours, a mixture of mPEG₃₆-NH₂ (0.4 equiv.) in DMF and DIPEA (3.0 equiv.) were added and stirred at 25 °C for 65 hours. A reaction conversion ratio was measured by liquid chromatography mass spectrometry (LCMS), and the reaction conversion ratio calculated by standardization was 20%.

### Example 87: Preparation of FP-3a (monoPEGylation)

A mixture of Compound F-3a (1.0 equiv.) in DMF was stirred at 25 °C for 15 minutes. A solution of DCC (1.1 equiv.) and HOBt (1.1 equiv.) in DMF was added dropwise to the above mixture. After stirring at 25 °C for 2 hours, a mixture of mPEG₃₆-NH₂ (0.4 eq.) in DMF was added and stirred at 25 °C for 24 hours. A reaction conversion ratio was measured by liquid chromatography mass spectrometry (LCMS), and the reaction conversion ratio calculated by standardization was 26%.

### Example 88: Preparation of FP-3a (monoPEGylation)

A mixture of Compound F-3a (1.0 equiv.) in DMF was stirred at 25 °C for 15 minutes. A solution of DCC (1.0 equiv.) and HOBt (1.0 equiv.) in DMF was added dropwise to the above mixture. After stirring at 25 °C for 2 hours, a mixture of mPEG₃₆ -NH₂ (0.4 equiv.) in DMF and DIPEA (3.0 equiv.) were added and stirred in a blackout curtain at 25 °C for 16 hours. A reaction conversion ratio was measured by liquid chromatography mass spectrometry (LCMS), and the reaction conversion ratio calculated by standardization was 17%.

### Example 89: Preparation of FP-3a (monoPEGylation)

A mixture of Compound F-3a (1.0 equiv.) in DMSO was stirred at 25 °C for 15 minutes. A solution of BEP (0.9 equiv.) in DMSO was added dropwise to the above mixture. After stirring at 25 °C for 2 hours, a mixture of mPEG₃₆-NH₂ (0.4 equiv.) in DMSO and DIPEA (3.0 equiv.) were added and stirred at 25 °C for 1 hour. A reaction conversion ratio was measured by liquid chromatography mass spectrometry (LCMS), and the reaction conversion ratio calculated by standardization was 43%.

### Example 90: Preparation of Compound FdP-3a (biPEGylation)

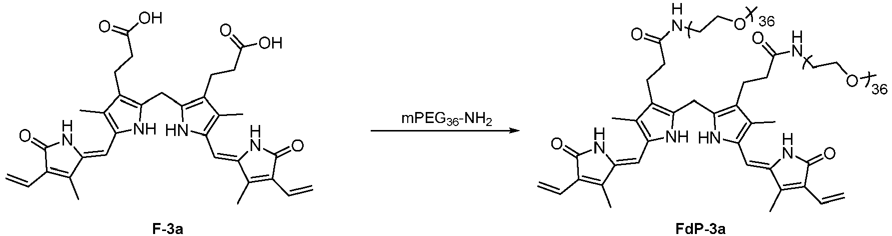

A mixture of Compound F-3a (200 mg, 0.34 mmol, 1.0 equiv.), which corresponds to the compound represented by Formula 3 herein prepared above, as well as HOBt (138.67 mg, 1.03 mmol, 3.0 equiv.), mPEG₃₆-NH₂ (1.38 g, 0.86 g, 2.5 equiv.) and EDCI (196.73 mg, 1.03 mmol, 3.0 equiv.) in DMSO (10 mL) was stirred at 25 °C for 30 minutes. A mixture of DIPEA (0.18 mL, 1.03 mmol, 3.0 equiv.) in DMSO (10 mL) was added to the above mixture, and stirred at 25 °C for 12 hours under nitrogen condition. Chloroform (400 mL) and water (300 mL) were added to the mixture, followed by extraction and washing. The combined organic layer was washed with brine (100 mL × 2), dried over anhydrous Na₂SO₄, filtered and concentrated under reduced pressure condition. The residue was purified by prep-HPLC to yield Compound FdP-3a (358.31 mg, 0.94 mmol, yield: 27%), which was bi-PEGylated from the compound corresponding to the compound represented by Formula 3 herein, as a red solid.

### Example 91: Preparation of Compound FdP-3a (biPEGylation)

A mixture of Compound F-3a (1.0 equiv.), which corresponds to the compound represented by Formula 3 herein prepared above, as well as HOBt (3.0 equiv.), mPEG₃₆-NH₂ (2.5 equiv.) and EDCI (3.0 equiv.) in DMSO was stirred at 25 °C for 30 minutes. A mixture of DIPEA (3.0 equiv.) in DMSO was stirred at 25 °C for 42 hours under nitrogen condition. A reaction conversion ratio was measured by liquid chromatography mass spectrometry (LCMS), and the reaction conversion calculated by standardization was 36%.

### Example 92: Preparation of Compound FdP-3a (biPEGylation)

A mixture of Compound F-3a (1.0 equiv.), which corresponds to the compound represented by Formula 3 herein prepared above, as well as mPEG₃₆-NH₂ (2.5 equiv.) and CDI (2.5 equiv.) in DMSO was stirred at 25 °C for 30.5 hours under nitrogen condition. A reaction conversion ratio was measured by liquid chromatography mass spectrometry (LCMS), and the reaction conversion ratio calculated by standardization was 34%.

### 5. Coupling of PEGylated Formula 1 Compound and Formula 2 Compound

After PEGylating Compound D of Example 2, Ea of Example 8 was coupled to yield FP-3a, which is a PEGylated compound of the compound represented by Formula 3.

### Example 93: PEGylation of Compound D

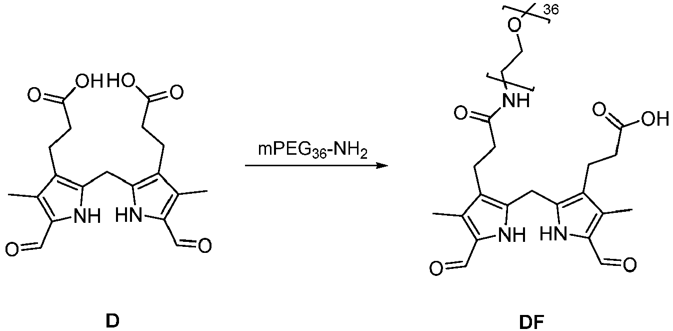

Compound D (500 mg, 1.34 mmol, 1.0 equiv.) was dissolved in DMSO (20 mL) and stirred at 25 °C for 15 minutes. A mixture of CDI (325 mg, 2.00 mmol, 1.5 equiv.) in DMSO (10 mL) was added dropwise to the above mixture. After stirring at 25 °C for 2 hours, a mixture of mPEG₃₆-NH₂ (2.16 g, 1.34 mmol, 1.0 equiv.) in DMSO (10 mL) was added to the reaction mixture and stirred at 25 °C for 4 hours. The organic layer was extracted using chloroform (250 mL) and water (50 mL × 2). The combined organic layer was washed with brine (50 mL × 2), dried over anhydrous Na₂SO₄, filtered and concentrated under reduced pressure condition. The residue was purified through a reversed-phase column to yield Compound DF (270 mg, 0.137 mmol, yield: 10%), which was monoPEGylated from the compound corresponding to the compound represented by Formula 1 herein, as a bright yellow solid.
C₉₂H₁₆₉N₃O₄₁ m/z [M]⁺ = 1972

### Example 94: Preparation of Compound FP-3a from Compound DF

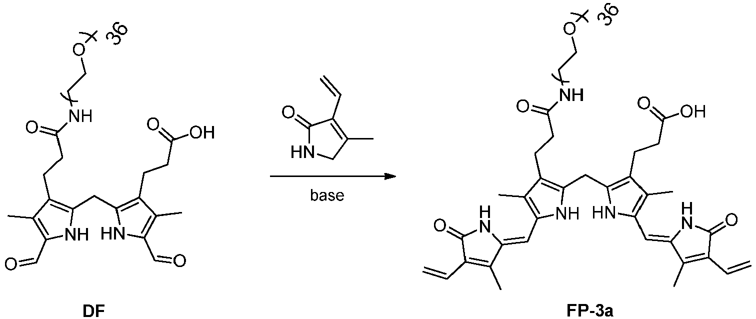

Piperidine (116 mg, 1.37 mmol, 10.0 equiv.) was added to a mixture of Compound Ea (51 mg, 0.41 mmol, 3.0 equiv.) and Compound DF (270 mg, 0.14 mmol, 1.0 equiv.) prepared above in 1,4-dioxane (10 mL), and stirred at 100 °C for 16 hours under nitrogen condition. The mixture was concentrated under reduced pressure condition. The residue was purified through prep-HPLC to yield Compound FP-3a (16 mg, 2 steps yield: 0.5%), which was monoPEGylated from the compound corresponding to the compound represented by Formula 3 herein, as a brown solid.
C₁₀₆H₁₈₃N₅O₄₁ m/z [M]⁺ = 2182

## Claims

1. A method for synthesizing bilirubin, comprising preparing a compound represented by Formula 3 below by coupling a compound represented by Formula 1 below with a compound represented by Formula 2 below: 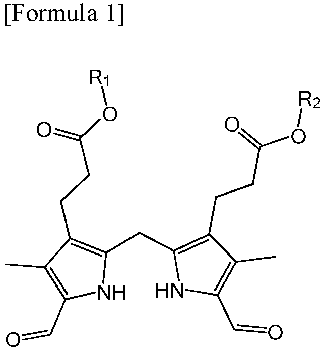 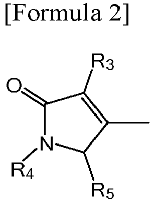 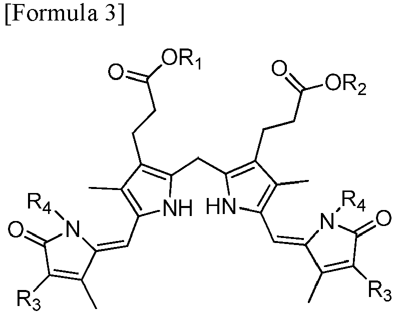 (wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₃ is hydrogen, a vinyl group, an acetyl group, or an ethyl group substituted with a hydroxyl group, selenide or sulfide; R₄ is hydrogen or a nitrogen protective group; and R₅ is hydrogen, a tosyl or mesyl group).

2. The method according to claim 1, further comprising reacting the compound represented by Formula 3 with polyethylene glycol (PEG).

3. The method according to claim 1, wherein the compound represented by Formula 1 is reacted with polyethylene glycol (PEG) and then coupled with the compound represented by Formula 2.

4. The method according to claim 1, further comprising dimerizing a compound represented by Formula 6 below to prepare the compound represented by Formula 1: 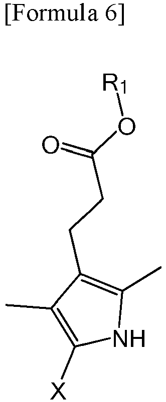 (wherein, R₁ is the same as R₁ in Formula 1, X is an arylalkyl ester group having 8 to 20 carbon atoms, -CH₂OH, -COOH, halogen atom or hydrogen).

5. The method according to claim 1, further comprising performing cyclization of a compound represented by Formula 8 below to prepare the compound represented by Formula 2: 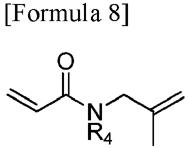 (wherein, R₄ is the same as R₄ in Formula 2).

6. The method according to claim 1, further comprising reducing the acetyl group in a compound represented by Formula 11 below to prepare the compound represented by Formula 2: 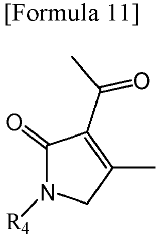 (wherein, R₄ is the same as R₄ in Formula 2).

7. The method according to claim 1, further comprising dehydrating the hydroxyl group in a compound represented by Formula 12 below to prepare the compound represented by Formula 2: 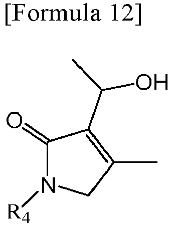 (wherein, R₄ is the same as R₄ in Formula 2).

8. The method according to claim 1, further comprising performing cyclization of a compound represented by Formula 13 below to prepare the compound represented by Formula 2: 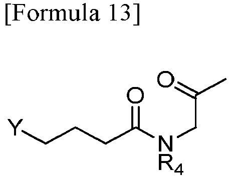 (wherein, Y is selenide, and R₄ is the same as R₄ in Formula 2).

9. The method according to claim 1, further comprising oxidizing a compound represented by Formula 14 below to prepare the compound represented by Formula 2: 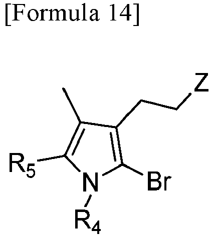 (wherein, Z is sulfide, R₄ and R₅ are the same as R₄ and R₅ in Formula 2).

10. The method according to claim 1, further comprising preparing bilirubin from the compound represented by Formula 3.

11. The method according to claim 1, wherein the step of preparing a compound is carried out in the presence of a base selected from the group consisting of: piperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidine-3-ol, piperazine, 2,6-dimethylpiperazine, 1-benzyl piperazine, 1-isopropyl piperazine, 2-ethyl piperazine, morpholine, 4-methyl morpholine, 2,6-dimethyl morpholine, ethyl morpholine, azepane, 2-methyl azepan, 4-methyl azepane, 2,2,7,7-tetramethyl azepane, 1,2,2-trimethyl azepane, 1,2-dimethylazepane, 2,7-dimethyl azepane, methyl azepane-4-carboxylate, azocane, 2-methyl azocane, 1,2-dimethyl azocane, 1,2,2-trimethyl azocane, methyl azocane-2-carboxylate, 1-methyl azocane and 2(2-methylphenyl)azocane.

12. The method according to claim 1, wherein the step of preparing a compound is carried out in the presence of a solvent selected from the group consisting of: water, alcohols, ethers, ketones, aliphatic hydrocarbons, aromatic hydrocarbons, halogenated hydrocarbons, alkoxies, nitriles and amides.

13. The method according to claim 1, wherein the step of preparing a compound is carried out at a temperature of -20 to 200 °C.

14. The method according to claim 1, wherein the step of preparing a compound is carried out for 0.5 to 120 hours.

15. The method according to claim 11, wherein the base is added in an amount of 2 to 20 moles based on 1 mole of the compound represented by Formula 1.
